# EUROPEAN PATENT SPECIFICATION

(11) **EP 0 612 321 B1**
(45) Date of publication and mention of the grant of the patent: **25.08.1999**
(21) Application number: 92922090.3
(22) Date of filing: 08.10.1992
(51) Int. Cl.: C07D 471/04, C07D 237/32, A61K 31/50, C07D 213/79, C07C 69/76

(54) **PYRIDO PYRIDAZINONE AND PYRIDAZINTHIONE COMPOUNDS WITH PDE IV INHIBITING ACTIVITY**
PYRIDO PYRIDAZINON UND PYRIDAZINTHIONVERBINDUNGEN MIT PDE IV INHIBIERENDER WIRKUNG
COMPOSES PYRIDO PYRIDAZINONES ET PYRIDAZINTHIONES A ACTIVITE INHIBANT LA PHOSPHODIESTERASE IV

(30) Priority: 09.10.1991 US 775679
(43) Date of publication of application: 31.08.1994
(73) Proprietor: SYNTEX (U.S.A.) INC., Palo Alto California 94304 (US)
(72) Inventor: WILHELM, Robert, Stephen, Mountain View, CA 94040 (US); LOE, Bradley, Earl, Santa Cruz, CA 95050 (US); DEVENS, Bruce, Henry, Palo Alto, CA 94301 (US); ALVAREZ, Robert, Menlo Park, CA 94024 (US); MARTIN, Michael, Grant, San Francisco, CA 94131 (US)
(74) Representative: Witte, Hubert, Dr.
(86) International application number: US9208398
(87) International publication number: WO9307146

(56) References cited:
- EP-A- 0 407 808
- WO-A-91/12251
- NAUNYN-SCHMIEDEBERG S ARCHIVES OF PHARMACOLOGY vol. 342, no. 2, 1990, BERLIN, DE pages 221 - 227 GALVAN, M., ET AL. 'Actions of the phophodiesterase inhibitor zardaverine on guinea-pig ventricular muscle'
- TETRAHEDRON, vol. 45, no. 17, 1989, OXFORD GB pages 5539 - 5548 CSAMPAI, A., ET AL. 'Bromoalkylphthalazinones and isomeric oxazolinium salts as intermediates and synthons'
- TETRAHEDRON, vol. 40, no. 15, 1984, OXFORD GB pages 2983 - 2984 FEKRY ISMAIL, M., ET AL. 'New convenient one-step synthesis of 4-arylphthalaz-1-ones'
- JOURNAL OF ORGANIC CHEMISTRY vol. 56, no. 17, 16 August 1991, EASTON US pages 5218 - 5221 JOHNSON, R.E. ET AL. 'Synthesis of alpha-substituted 1,2- benzenedimethanamines'
- CHIMICA THERAPEUTICA vol. VI, no. 2, 1971, FR pages 109 - 115 WERMUTH, C.G., ET AL. 'Dérives pyridaziniques présentant un intérêt thérapeutique.'
- CHEMICAL ABSTRACTS, vol. 108, 1988, Columbus, Ohio, US; abstract no. 131719n, BANSAL, R.K. 'Synthesis of substituted phthalazinones' page 747 ; & Proc. Natl. Acad. Sci., India, Sect A 1986, 56(4), 384-7
- CHEMICAL ABSTRACTS, vol. 106, 1987, Columbus, Ohio, US; abstract no. 156382u, AFIFY, A.A. 'Synthesis and reactions of phthalazine derivatives' page 682 ; & J. Chem. Soc. Pak. 1986, 8(1), 53-7
- CHEMICAL ABSTRACTS, vol. 105, 1986, Columbus, Ohio, US; abstract no. 42152t, BANSAL, R.J. 'Mass spectral analysis of phthalazinones' page 678 ; & Chim. Acta Turc. 1984, 12(2), 365-8
- CHEMICAL ABSTRACTS, vol. 100, 1984, Columbus, Ohio, US; abstract no. 51532r, OISHI, E. 'Condensed pyridazines. II. Chemical properties of 5-phenylpyrido(2, 3-d)pyridazine 7-oxide' page 576 ; & Yakugaku Zasshi 1983, 103(6), 631-43
- CHEMICAL ABSTRACTS, vol. 111, 1989, Columbus, Ohio, US; abstract no. 39286h, YAKOLEV, S.V. ET AL. 'Reaction of 2-aroylbenzoic acids with 4-nitro and 2,4- dinitrohydrazine' page 593 ; & Zh. Org. Khim. 1988, 24(11), 2433-6
- CHEMICAL ABSTRACTS, vol. 83, 1975, Columbus, Ohio, US; abstract no. 178862j, FAHMY, A.F.M., ET AL 'Acid azides. III. New syntheses of benzimidazolones, phthlaz-4-ones, and benzoxazolones involving base-catalyzed Curtius rearrangement and displacement of acid azides' page 558 ; & Indian J. Chem. 1975, 13(7), 652-4
- MONATSHEFTE FUR CHEMIE vol. 121, 1990, WIEN AT pages 909 - 921 EPSZTAJN, J. ET AL. 'Application of organolithium and related reagents in synthesis.'
- CHEMICAL ABSTRACTS, vol. 80, 1974, Columbus, Ohio, US; abstract no. 59626p, MAREK, V., ET AL. 'Polarographic reduction' page 332 ; & Chem. Zvesti 1973, 27(6), 787-91
- JOURNAL OF ORGANIC CHEMISTRY vol. 44, no. 6, 1979, EASTON US pages 984 - 989 VIVEKANANDA BHATT M., ET AL. 'Aspects of tautomerism. 7. Study of keto participation in alkaline hydrolysis of normal esters of gamma-keto acids'
- JOURNAL OF THE CHEMICAL SOCIETY, SECTION B: PHYSICAL ORGANIC CHEMISTRY 1971, LETCHWORTH GB pages 145 - 148 BOWDEN, K. ET AL 'Reactions of carbonyl compounds in basic solutions. Part II. The mechanism od the alkaline hydrolysis of methyl 2-benzoylbenzoates'

## Description

### FIELD OF THE INVENTION

This invention relates to pyridopyridazinones and pyridazinthiones useful as anti-inflammatory agents, antasthmatic agents, immunosuppressive agents, anti-allograft rejection agents, anti-graft-vs-host disease agents, anti-autoimmune agents or analgetic agents, to their intermediates, to their preparation and to pharmaceutical compositions using the compounds of the invention.

### BACKGROUND INFORMATION

Cyclic 3', 5'-adenosine monophosphate (CAMP) modulates a variety of cellular and physiologic functions in mammals, such as, cell division, endocrine function, and the immune response. The level of CAMP is controlled, in part, by a class of enzymes called phospho-diesterases, which enzymatically degrade cAMP. There are five families of phosphodiesterase that are categorized according to their function. For instance, phosphodiesterase III (PDE III) is isolated from human platelet cells and modulates platelet aggregation. Another type of phosphodiesterase, (PDE IV), is found in various cells but it is the predominant form in human leukocytes. This enzyme modulates leukocyte activation and function associated with the immune response and inflammation. Both of these phospho-diesterases implement their control by modulating the cellular level of cAMP in their respective cells. Thus, inhibition of phosphodiesterases provides a method of modulating any cellular and bodily function that is controlled by cAMP.

Compounds that are nonspecific phosphodiesterase inhibitors are known, i.e., these compounds inhibit all or multiple types of phosphodiesterases. [*See*, Beavo, J.A. and D.H. Reifsyder, *Trends in Pharm. Science,* 11:150-155 (1990); and Nicholson, C.D., R.A.J. Challiss and M. Shahid, *Trends in Pharm. Science,* 12:19-27 (1991).]

Nonspecific phosphodiesterase inhibitors are of limited value because of numerous side-effects. Since cAMP is involved in so many functions throughout the body, a nonspecific phosphodiesterase inhibitor has the potential to alter all of the functions modulated by cAMP.

Csampai et al. [*Tetrahedron* 45:5539-5548 (1989)], Ismail et al. [*Tetrahedron* 40:2983-2984 (1984)], Johnson et al. [*J. Org. Chem.* 56:5218-5221 (1991)], Jakolev et al. [*Zh. Org. Khim.* 24:2433-2436 (1988)] and Wermuth et al. [*Chimie Therapeutique* 6:109-115 (1971)] describe the preparation of phthalazinones and arylphthalazones as intermediates and synthons. Additional phthalazones have been described by Fahmy et al. [*Indian J. Chem.* 13:652-654 (1975)]. International Patent Application WO 91/12251 refers to fused heterocyclic compounds with antiasthmatic activity. Further examples for the synthesis and reactions of substituted phthalazinones and their spectral analysis are given by Bansal et al. [*Proc. Natl. Acad. Sci., India, Sect A* 56:384-387 (1986); Chim. Acta Turc. 12:365-368 (1984)] and Afify et al. [*J. Chem. Soc. Pak.* 8:53-57 (1986)].

Oishi et al. [*Yakugaku Zasshi,* 103:631-643 (1983)] describe the chemical properties of phenylpyridopyridazine-7-oxide and Epsztajn et al. [*Monatshefte für Chemie* 121:909-921 (1990)] disclose the preparation of 3-benzoylated picoline and isonicotine acids. Marek et al. [*Chem. Zvesti* 27:787-791 (1973)] disclose the polarographic reduction of carboxybenzophenone derivatives and Bhatt et al. [*J. Org. Chem.* 44:948-989 (1979)] and Bowden et al. [*J. Chem. Soc. Section B, Phys. Org. Chem.* 145-148 (1971)] the alkaline hydrolysis of substituted benzoylbenzoates. European Patent Application Publ. No. 407808 refers to a catalyst and processes for the polymerization of olefins.

It has been surprisingly discovered that certain pyridopyridazinones are potent selective inhibitors of phosphodiesterase IV (PDE IV). These compounds are well suited for use as a treatment for any disorder in which PDE IV function plays a role, such as where leukocyte activation or function is involved.

In particular, these compounds are especially well suited for use as anti-inflammatory agents, antasthmatic agents, immunosuppressive agents, anti-allograft rejection agents, anti-graft-vs-host disease agents or anti-autoimmune disease agents.

### SUMMARY OF THE INVENTION

One aspect of the present invention relates to pyrido-pyridazinones and pyridazinthiones, i.e., the compounds of Formula I: 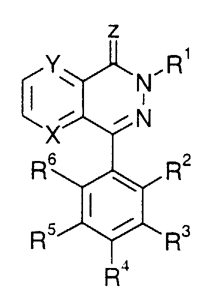 wherein:
- X and Y: are carbon or nitrogen, provided both are not the same;
- Z: is oxygen or sulfur;
- R¹: is hydrogen; phenyl or naphthyl, optionally mono or disubstituted with hydroxy, C₁₋₉ alkyl, C₁₋₉ alkoxy, trifluoromethyl, nitro or cyano; 5- or 6-membered heteroaryl containing 1 to 3 heteroatoms; 4-pyridylmethyl, 3-pyridylethyl, 2-pyridylmethyl, 2-pyridylpropyl, 3-thienylmethyl or 2-thienylethyl.
- R², R³, R⁴, R⁵ and R⁶: are independently selected from hydrogen, C₁₋₉ alkyl, halo, carboxy, C₁₋₉ alkoxycarbonyl, methylthio, trifluoromethyl, cyano, nitro and carbamoyl of the formula -C(O)NR'R" where R' and R" are independently hydrogen or C₁₋₉ alkyl;
or a pharmaceutically acceptable ester or salt thereof.

In another aspect, the invention relates to a pharmaceutical composition containing a therapeutically effective amount of the above-mentioned compounds or pharmaceutically acceptable esters or salts thereof admixed with at least one pharmaceutically acceptable excipient.

In still another aspect, the invention relates to a method of use as an anti-inflammatory, antasthmatic, immunosuppressive, anti-allograft rejection, anti-graft-vs-host rejection, autoimmune disease, or analgetic agent, by administering to a mammal in need of such treatment a therapeutically effective amount of a compound of Formula I or a pharmaceutically acceptable ester or salt thereof.

Yet another aspect of the invention relates to the treatment of the above conditions or diseases by the selective inhibition of phosphodiesterase (PDE) IV.

Yet another aspect of the invention relates to precursors for making the compounds of Formula I and the pharmaceutically acceptable salts and esters thereof, represented by Formula II: 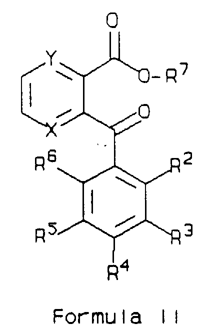 wherein:
- X, Y, R², R³, R⁵, R⁶: are as defined in Formula I R⁴ is hydrogen, C₁₋₉ alkyl, iodo, bromo, chloro, carboxy, C₁₋₉ alkyl carbonyl, nitro or carbamoyl of the formula -C(O)NR'R" where R' and R" are independently hydrogen of C₁₋₉ alkyl; and
- R⁷: is C₁₋₉ alkyl; provided that when X is carbon, Y is nitrogen and R⁷ is methyl, R², R³, R⁴, R⁵ and R⁶ are not all hydrogen;
or a pharmaceutically acceptable ester or salt thereof.

Another aspect of the invention relates to processes for making the compounds of Formula I and the pharmaceutically acceptable salts and esters thereof.

For example, a compound of Formula I (where Z is oxygen) is made by reacting a compound of Formula II with a suitably substituted hydrazine. Or, a compound of Formula I (where Z is oxygen) is treated with a thiation reagent for ketones, e.g., Lawesson's Reagent, to give the corresponding compound of Formula I where Z is sulfur.

Another aspect of the invention relates to processes for making the compounds of Formula I (where R¹ is other than hydrogen by reacting a compound of Formula I (where R¹ is hydrogen) with a suitably substituted alcohol.

In another aspect, this invention provides compositions useful in the treatment of inflammatory, asthmatic, allograft rejection, graft-vs-host or autoimmune conditions or diseases, or pain in mammals through its use as an anti-inflammatory, antasthmatic, immunosuppressive, anti-allograft rejection, anti-graft-vs-host rejection, autoimmune disease, or analgetic agent, wherein the composition comprises a therapeutically effective amount of a compound of Formula I as described above and a pharmaceutically acceptable excipient.

### DETAILED DESCRIPTION

### DEFINITIONS AND GENERAL PARAMETERS

The following definitions are set forth to illustrate and define the meaning and scope of the various terms used to describe the invention herein.

The term "lower alkylene" refers to a biradical branched or unbranched saturated hydrocarbon chain containing 1 to 6 carbon atoms, such as methylene, ethylene, propylene, isopropylene and butylene.

The 5- or 6-membered heteroaryl ring which contains 1 to 3, identical or different hetero atoms such as nitrogen, oxygen or sulfur. Typical examples are: pyridyl, pyridyl-N-oxide, thienyl, furyl, pyrrolyl, pyrrolidinyl, imidazolyl, pyrimidinyl, pyrazinyl, pyridazinyl, oxazolyl, isoxazolyl, thiazolyl, isothiazolyl, pyrazolyl, pyridonyl, and the like.

The term "halo" refers to fluoro, bromo, chloro, iodo.

The term "pharmaceutically acceptable salt" refers to any salt derived from an inorganic or organic acid or base. The term "pharmaceutically acceptable anion" refers to the anion of such acid addition salts. The term "pharmaceutic- ally acceptable cation" refers to the cation of such base addition salts. The salt, anion and/or the cation are chosen not to be biologically or otherwise undesirable.

The anions are derived from inorganic acids, such as hydrochloric acid, hydrobromic acid, sulfuric acid (giving the sulfate and bisulfate salts), nitric acid, phosphoric acid and the like, and organic acids such as acetic acid, propionic acid, hexanoic acid, heptanoic acid, cyclopentanepropionic acid, glycolic acid, lactic acid, pyruvic acid, oxalic acid, malic acid, malonic acid, succinic acid, maleic acid, fumaric acid, tartaric acid, citric acid, benzoic acid, o-(4-hydroxybenzoyl)benzoic acid, benzenesulfonic acid, p-chlorobenzenesulfonic acid, 2-naphthalenesulfonic acid, cinnamic acid, mandelic acid, methanesulfonic acid, ethanesulfonic acid, 1,2-ethane disulfonic acid, 2-hydroxyethanesulfonic acid, salicylic acid, p-toluenesulfonic acid and the like.

The cations are derived from bases, such as alkaline earth hydroxides, including calcium hydroxide, potassium hydroxide, potassium carbonate, sodium hydroxide, sodium hydride, lithium hydroxide and the like, preferably sodium hydroxide.

The term "pharmaceutically acceptable esters" and "pharmaceutically acceptable ethers" refer to those compounds of Formula I where an oxygen or a nitrogen has been modified, e.g., acylated by the addition of the group -C(=O)-W, wherein W is an alkyl group containing 1 to 20 carbon atoms including adamantyl, aryl, amino, alkylamino, dialkylamino, an alkoxy group containing 1 to 20 carbon atoms, -CH₂-O-CH₃, or -CH₂-NH₂. This invention contemplates those compounds of Formula I which are esters as described herein and at the same time are the pharmaceutically acceptable acid addition salts thereof.

The terms "inert organic solvent" or "inert solvent" refer to a solvent inert under the conditions of the reaction being described in conjunction therewith [including, for example, benzene, toluene, acetonitrile, tetrahydrofuran ("THF"), dimethylformamide ("DMF"), chloroform, methylene chloride (or dichloromethane), diethyl ether, pyridine and the like]. Unless specified to the contrary, the solvents used in the reactions of the present invention are inert organic solvents.

As used herein, the term "autoimmune disease" refers to disorders wherein the immune system of a mammal mounts a humoral or cellular immune response to the mammal's own tissue or to antigenic agents that are not intrinsically harmful to the mammal, thereby producing tissue injury in such a mammal. Examples of such disorders include, but are not limited to, systemic lupus erythematosus, rheumatoid arthritis and type I diabetes.

As used herein, the term "allograft rejection" refers to the humoral or cellular immune response mounted by the immune system of a mammal after it has received a histo-incompatible tissue graft from another mammal of the same species, thereby producing tissue injury in such a mammal.

As used herein, the term "graft-vs-host disease" refers to the immune response that originates from transplanted graft tissue, in particular, transplanted bone-marrow tissue, and that is directed towards the host tissue, thereby producing tissue injury in the host.

As used herein, the term "treatment" or "treating" means any treatment of a disease in a mammal, including:
(i) preventing the condition or disease, that is, avoiding any clinical symptoms of the disease;
(ii) inhibiting the condition or disease, that is, arresting the development or progression of clinical symptoms; and/or
(iii) relieving the condition or disease, that is, causing the regression of clinical symptoms.

As used herein, the term "therapeutically effective amount" refers to that amount of a compound of Formula I which, when administered to a mammal in need thereof, is sufficient to effect treatment (as defined above) as an anti-inflammatory, antasthmatic, immunosuppressive, anti-allograft rejection, anti-graft-vs-host disease, autoimmune disease or analgetic agent(s). What amount constitutes a "therapeutically effective amount" will vary depending on the compound, the condition and its severity, and the mammal to be treated, but may be determined routinely by one of ordinary skill in the art having regard to his own knowledge and to this disclosure.

As used herein, the term "q.s." means adding a quantity sufficient to achieve a stated function, e.g., to bring a solution to a desired volume (e.g., 100 mL).

Unless specified to the contrary, the reactions described herein take place at atmospheric pressure over a temperature range from about -100°C to about 200°C, more preferably from about 10°C to about 50°C, and most preferably at about room (or "ambient") temperature, e.g., about 20°C.

Isolation and purification of the compounds and intermediates described herein can be effected, if desired, by any suitable separation or purification procedure such as, for example, filtration, extraction, crystallization, column chromatography, preparative high pressure liquid chromatography (preparative HPLC), thin-layer chromatography or thick-layer chromatography, or a combination of these procedures. Specific illustrations of suitable separation and isolation procedures can be had by reference to the examples hereinbelow. However, other equivalent separation or isolation procedures can, of course, also be used.

### Naming of Compounds

For the compounds of the instant invention containing a pyridopyridazinone ring moiety, where X is nitrogen, Y is carbon, and Z is oxygen (i.e., pyrido[2,3-d]pyridazin-5-one compounds), the following numbering system will be used: 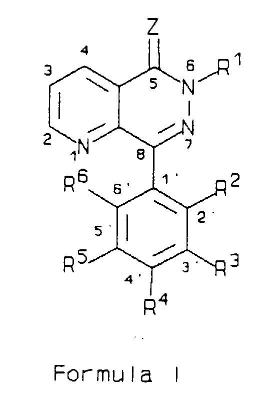 Representative compounds are named in the following examples.

The compound of Formula I where X is nitrogen, Y is carbon, Z is oxygen, R¹ is 4-pyridyl-N-oxide-methyl, R³ is chloro, R², R⁴, R⁵ and R⁶ are hydrogen, can be named 6-(4-pyridyl-N-oxide-methyl)-8-(3-chlorophenyl)-pyrido[2,3-d]pyridazin-5-one.

The compound of Formula I where X is nitrogen, Y is carbon, Z is sulfur, R¹ is benzyl, R³ is chloro, R², R⁴, R⁵ and R⁶ are hydrogen, can be named 6-benzyl-8-(3-chlorophenyl)pyrido[2,3-d]pyridazin-5-thione.

For the compounds of the instant invention containing a pyridopyridazinone ring moiety, where X is carbon, Y is nitrogen, and Z is oxygen (i.e., pyrido[2,3-d]pyridazin-8-one compounds), the following numbering system will be used:

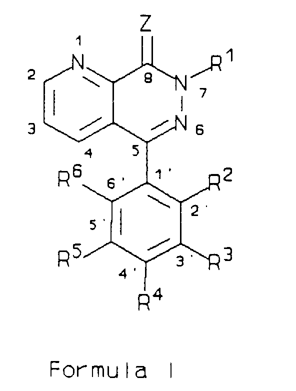

The compound of Formula I where X is carbon, Y is nitrogen, Z is oxygen, R¹ is benzyl, R³ is nitro, R², R⁴, R⁵ and R⁶ are hydrogen, can be named 7-benzyl-5-(3-nitrophenyl)pyrido[2,3-d]pyridazin-8-one.

### SYNTHESIS OF THE COMPOUNDS OF FORMULAE I AND II

The compounds of Formulae I and II are synthesized as described with reference to Reaction Scheme A.

Reaction Scheme A illustrates the preparation of novel pyridopyridazinones (Formula I where X is nitrogen, Y is carbon, and Z is oxygen) and the corresponding intermediate pyridoarylketoesters (Formula II).

Reaction Scheme B illustrates a preparation for the intermediate pyridoarylketoesters (Formula II where X and Y are carbon or nitrogen, provided both are not the same, using an organolithium, organocadmium or Grignard reagent.

Reaction Scheme C illustrates an alternative preparation of novel pyridopyridazinones (Formula I where Z is oxygen) from pyridopyridazinones, where R¹ is hydrogen.

Reaction Scheme D illustrates a preparation for the intermediate pyridoarylketoesters (Formula II where X and Y are carbon or nitrogen, provided both are not the same, and R³ is nitro).

Reaction Scheme E illustrates a preparation of novel pyridyl-N-oxide-methyl substituted pyridopyridazinones (Formula I) from pyridopyridazinones (Formula I), where R¹ is hydrogen.

Reaction Schemes F and G illustrate an alternative preparation of novel optionally substituted compounds of Formula I, where X is nitrogen, Y is carbon, and Z is oxygen.
Shown in Reaction Scheme F is the preparation of optionally substituted nitrophenyl heteroaryl hetero-oxide pyridopyridazinones (compounds of Formula I, where X is nitrogen, Y is carbon, Z is oxygen, R¹ is heteroaryl hetero-oxide, and R³ is nitro).
Shown in Reaction Scheme G is the preparation of optionally substituted chlorophenyl heteroaryl hetero-oxide pyridopyridazinones (Formula I, where X is nitrogen, Y is carbon, Z is oxygen, R¹ is heteroaryl hetero-oxide, and R³ is chloro).

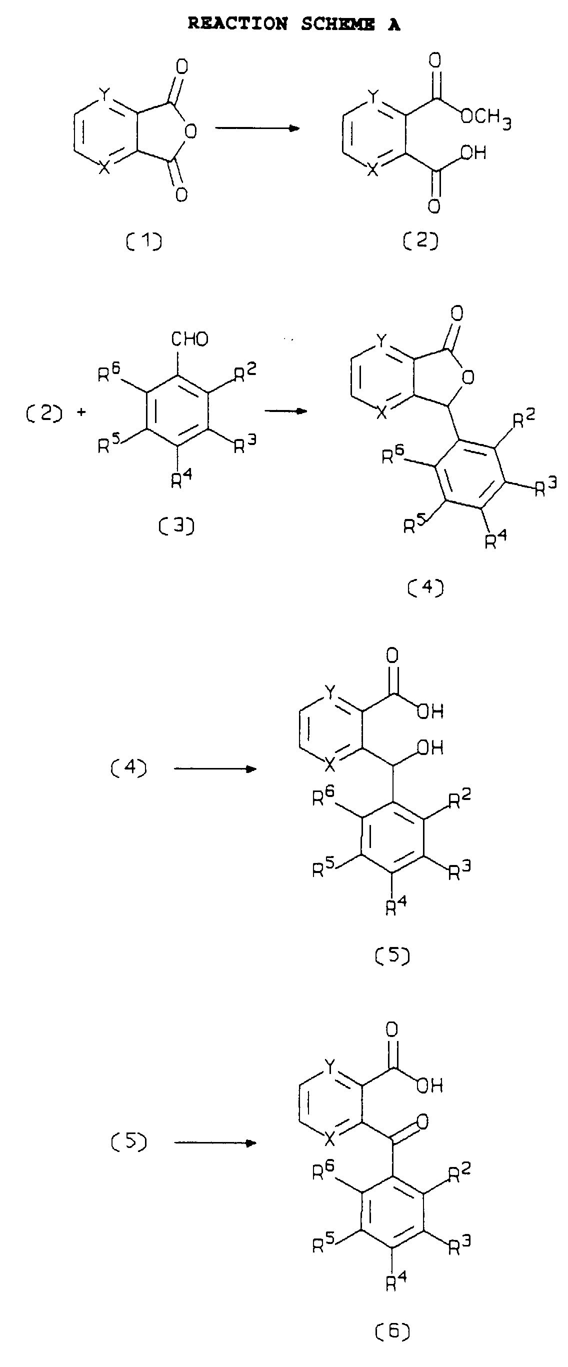

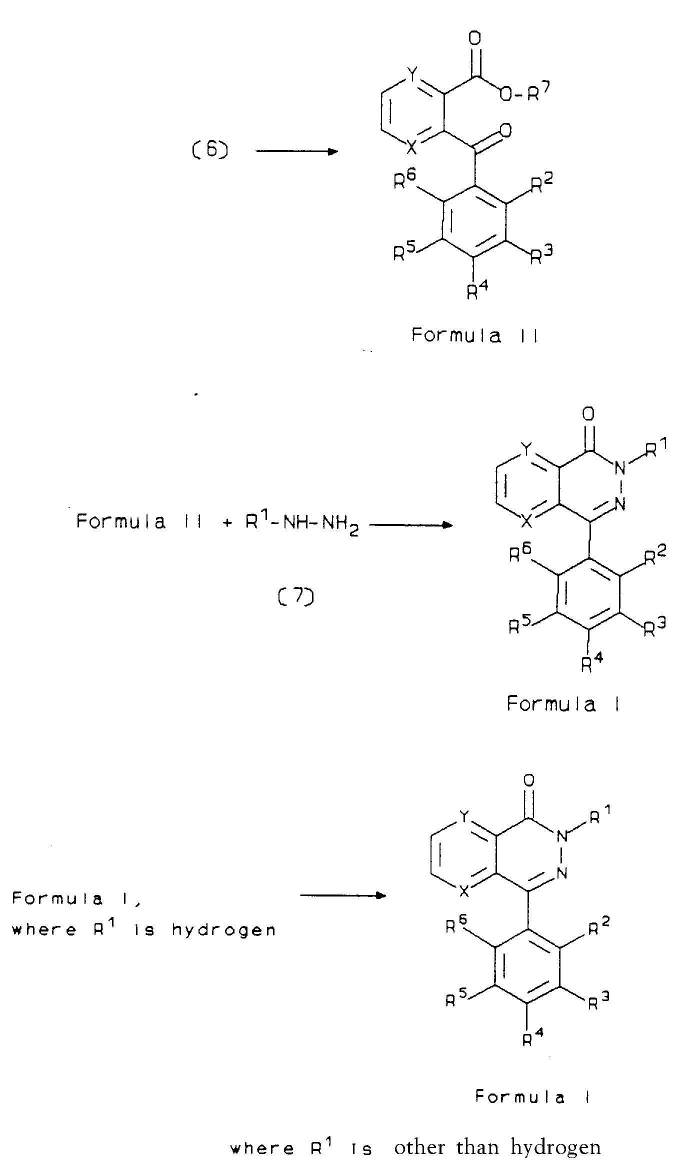

### STARTING MATERIALS

Referring to Reaction Scheme A, most of the compounds of Formula 1 are commercially available from Aldrich Chemicals and ICN Chemicals.
Similarly, many of the substituted hydrazines (Formula 7) are commercially available from Aldrich Chemicals, those that are not can be prepared by following the disclosures of S.R. Sandler and W. Karo, "*Organic Functional Group Preparations"* 2nd Ed. 1983, Vol 12-I, Academic Press, New York.

### PREPARATION OF FORMULA 2

A pyridodicarboxylic anhydride (Formula 1) is dissolved in a solvent [e.g., an alcohol (methanol or ethanol), preferably methanol] and heated for a period of about 2 to 20 minutes, preferably about 10 minutes at a temperature of about 80 to 120°C, preferably about 100°C.
The desired product is taken directly to the next step without purification.

### PREPARATION OF FORMULA 4

An optionally substituted pyridoacidester (Formula 2) is added to about 5.0 molar equivalents of an optionally substituted benzaldehyde (Formula 3) and about 5.0 molar equivalents of a solvent, such as p-cymene, dibromobenzene, nitrotoluene, bromoanisole or xylene, preferably *p*-cymene. The mixture is heated to a temperature of about 150°C to 210°C, preferably about 180°C for a period of about 2 to 6 hours, preferably about 4 hours. The solution is cooled and the excess benzaldehyde and solvent are removed yielding the desired optionally substituted pyridolactone (the compound of Formula 4). The desired compound can be purified by silica gel chromatography or the like, however, preferably the desired compound is taken without purification directly to the next step.

### PREPARATION OF FORMULA 5

An optionally substituted pyridolactone (Formula 4) is suspended in a basic solution (e.g., potassium hydroxide, sodium hydroxide or ammonium hydroxide in a solvent such as, methanol or ethanol, preferably potassium hydroxide in methanol at a ratio of about 4.5 grams/100ml) and refluxed for a period of about 3 to 9 hours, preferably about 6 hours. The mixture is cooled and the solvent removed yielding a pyridobenzylhydroxy acid (Formula 5).

### PREPARATION OF FORMULA 6

A pyridobenzylhydroxy acid (Formula 5) is suspended in a solvent that is inert to oxidation agents (e.g., methylene chloride, chloroform or carbon tetrachloride, preferably methylene chloride). To this solution is added about 1.0 molar equivalent of an oxidation agent, such as pyridinium dichromate, pyridinium chlorochromate or manganese dioxide, preferably pyridinium dichromate. The solution is stirred at a temperature in the range of about 0°C to 50°C, preferably about 21°C (room temperature) for a period of about 9 to 27 hours, preferably about 18 hours, yielding the desired optionally substituted pyridobenzoylacid (Formula 6). The desired product can be isolated and purified, however, the product is preferably taken directly to the next step without purification.

### PREPARATION OF FORMULA II

A pyridobenzoylacid (Formula 6) is dissolved in a solvent (e.g., methylene chloride, chloroform or carbon tetrachloride, preferably methylene chloride) and cooled to a temperature in the range of about -20°C to 20°C, preferably about 0°C. About 1.2 molar equivalent of an esterification reagent such as, diazomethane, is added to the solution and stirred for a period of about 1 to 5 hours, preferably the reaction is monitored by TLC to completion (about 2 hours). The desired optionally substituted pyridobenzoylester (Formula II) is isolated and purified by the removal of the solvent followed by chromatography.

Alternatively, an optionally substituted pyridobenzoylester (Formula II) is prepared by dissolving a suitably substituted pyridobenzoylacid (Formula 6) in a solvent (e.g., methanol, ethanol or the like, preferably methanol) to which is added about 1 molar equivalent of a strong acid (e.g., concentrated H₂SO₄, HCl (gas), BF₃·Et₂O, preferably concentrated H₂SO₄).
The solution is stirred for a period of about 6 to 30 hrs, preferably about 18 hrs at the reflux temperature of the solvent used. The solution is cooled and made basic. The desired optionally substituted pyridobenzoylester (Formula II) is isolated and purified by chromatography.

As indicated the desired compound may be purified by silica gel chromatography or the like, however, preferably the desired compound is taken without further purification to the next step.

### PREPARATION OF FORMULA I

An optionally substituted pyridobenzoylester (Formula II) is suspended in a solvent (about 15 ml/mmole) (e.g., ethanol, methanol or the like, preferably ethanol). To this suspension is added about 2 molar equivalents of an unsubstituted or substituted hydrazine (Formula 7). The mixture is refluxed for a period of about 4 hours to 240 hours. The specific time is dependent on the substituent (i.e., R¹) of the hydrazine. Preferably, the reaction mixture is monitored by TLC for completion. The mixture is allowed to cool and filtered to give a crude product. The desired pyridobenzoylpyridazinones (Formula I) are purified and isolated following extraction and chromatography.

Other optionally substituted compounds of Formula I are prepared by suspending a suitably substituted compound of Formula I, where R¹ is hydrogen, in a solvent (about 10 to 100ml/mmole). To this suspension is added about 1 to 20 molar equivalents of a suitable base (e.g., sodium hydride, potassium carbonate, potassium iodide) and about 1 to 20 molar equivalents of a suitable alkylating agent (e.g., iodoethane, 4-picolyl chloride, 2-iodopropane). The mixture is refluxed under an inert atmosphere for about 6 to 48 hours, preferably about 18 hours, most preferably the reaction is monitored by TLC for completion. The mixture is allowed to cool and purified following standard chromatographic techniques yielding the desired pyridobenzoylpyridazinones (Formula I).

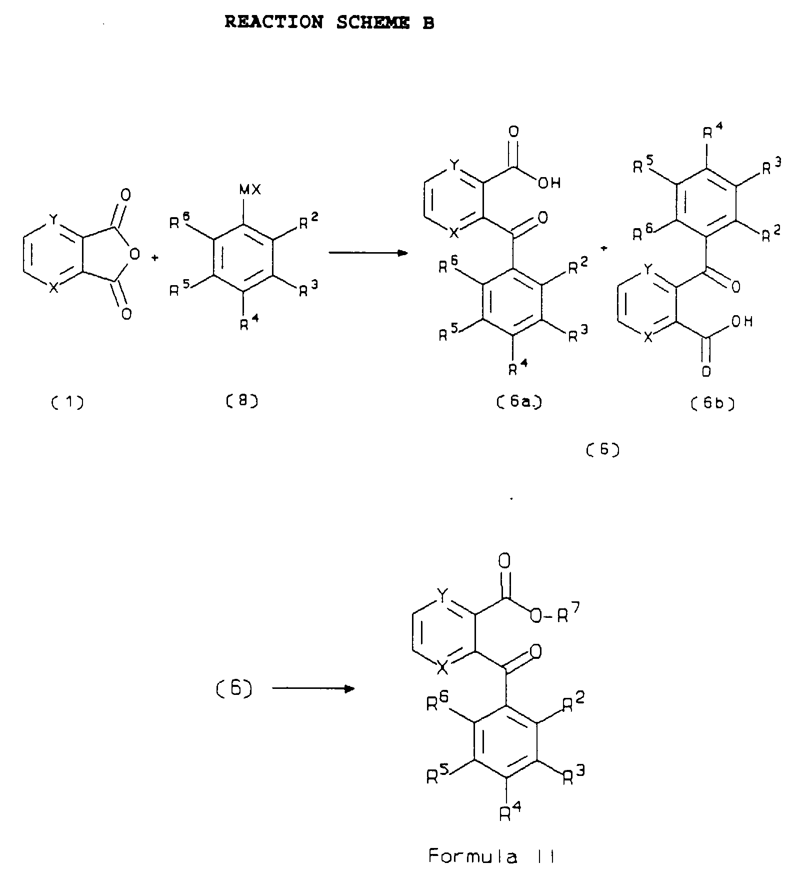

### ALTERNATE PREPARATION OF FORMULA II

Alternatively, the compounds of Formula II can be prepared using an organolithium or organocadmium reagent as illustrated in "Preparation of Aroylbenzoic Acid. Reaction of Aryllithium Reagents with Phthalic Anhydride", *J. Organic Chem.,* 41(7), 1268 (1976), or by Grignard reagent.

The Grignard preparation is illustrated in Reaction Scheme B (where M is Mg). A Grignard reagent is prepared by adding to a solution of an optionally substituted halogenated aryl compound (Formula 8) in a solvent (e.g., tetrahydrofuran or ethyl ether, preferably tetrahydrofuran) about 1 molar equivalent of magnesium followed by stirring for a period of 12 to 24 hours, preferably 18 hours, at a temperature in the range of about 0°C to 50°C, preferably room temperature under an inert atmosphere. The Grignard reagent (Formula 8) is added to a pyridodicarboxylic anhydride (Formula 1) in a solvent (e.g., tetrahydrofuran, methylene chloride, chloroform or ethyl ether, preferably tetrahydrofuran) at a temperature in the range of about 0°C to -150°C, preferably about -78°C in a gradual manner over a period of about 2 hours. After the addition, the solution is stirred for a period of about 15 minutes to 75 minutes, preferably about 45 minutes. An acid (e.g., 1N HCl) is added to the solution at a slight molar excess and stirred for a period of about 1 hour. In the case where Formula 1 is pyridodicarboxylic anhydride (i.e., where either X or Y are nitrogen), the preparation results in two positional isomers of the Formula 6, i.e. Formulae 6a and 6b. Depending on the desired product, i.e., Formula I where X is nitrogen and Y is carbon, or Formula I where X is carbon and Y is nitrogen, the suitable isomer of Formula 6 (Formulae 6a or 6b) is isolated and purified and used in the following preparations as the compound of Formula 6.

### PREPARATION OF FORMULA II

An optionally substituted pyridobenzoylester (Formula II) can be prepared from a pyridobenzoylacid (Formula 6) by following the procedures set forth in Reaction Scheme A

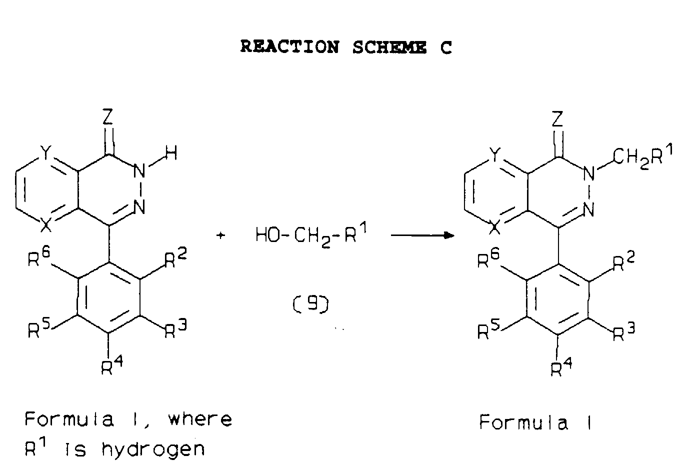

### PREPARATION OF FORMULA I WHERE R¹ IS LOWER ALKYL, ARYL OR HETEROCYCLE.

Alternatively, optionally substituted pyridopyridazinone compounds (Formula I, where R¹ is other than hydrogen) can be prepared from a pyridopyridazinone compound (Formula I) where R¹ is hydrogen by following Mitsunobu reaction conditions, as illustrated in O. Mitsunobu, *Synthesis,* 1, 1981, and incorporated herein by reference.

To a suspension of a pyridopyridazinone (Formula I) where R¹ is hydrogen in a solvent (e.g., tetrahydrofuran, diethyl ether, dioxane, preferably tetrahydrofuran) is added about 1 molar equivalent of an optionally substituted alcohol (Formula 9, where R¹ is other than hydrogen) and about 1.5 molar equivalent of a trisubstituted phosphine, preferably triphenylphosphine. To the solution is added about 1.5 molar equivalent of a disubstituted azodicarboxylate, such as diethyl azodicarboxylate (DEAD) or diisopropyl azodicarboxylate (DIAD), preferably diisopropyl azodicarboxylate in a gradual manner. The solution is stirred for a period of about 12 to 24 hours, preferably about 18 hours at a temperature in the range of about 0°C to 50°C, preferably about 21°C (room temperature). The solvent is removed and the desired optionally substituted pyridopyridazinone (Formula I, where R¹ is other than hydrogen) is isolated and purified by chromatography.

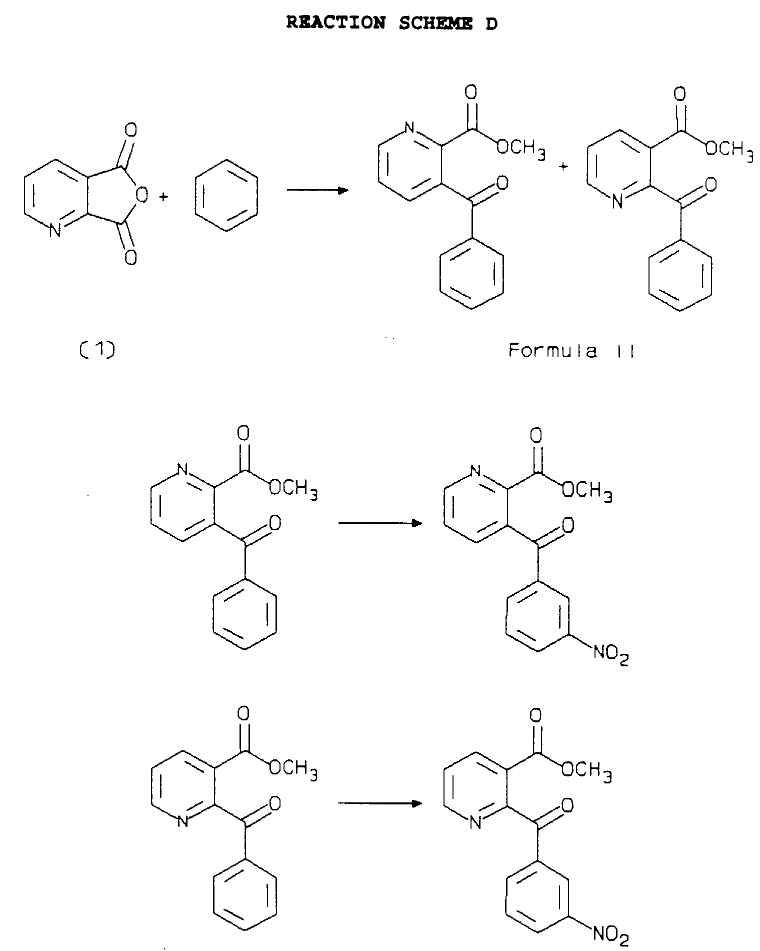

### PREPARATION OF FORMULA II WHERE X IS CARBON AND Y IS NITROGEN.

A pyridophenylketoester (i.e., Formula II where X and Y are carbon or nitrogen, provided both are not the same, and R², R³, R⁴, R⁵ and R⁶ are hydrogen) can be prepared following the procedures in U.S. Patent 4,806,535.

### PREPARATION OF FORMULA II WHERE X IS CARBON, Y IS NITROGEN AND R³ IS NITRO

A pyrido(3-nitrobenzoyl)ketoester (Formula II where X is carbon, Y is nitrogen and R³ is nitro) is prepared by dissolving a pyridophenylketoester in an acid (such as hydrochloric acid, nitric acid, sulfuric acid, preferably, sulfuric acid) and adding in a dropwise manner a solution of nitric acid and sulfuric acid (about 1 to 3 molar equivalent of nitric acid with about 2 ml of sulfuric acid per molar equivalent of nitric acid). The solution is stirred for about 10 to 60 minutes, preferably about 30 minutes, cooled and adjusted to a pH of about 7 to 8. The solvent is removed and the desired optionally substituted pyridopyridazinone (Formula I, where X is carbon, Y is nitrogen and R³ is nitro) is isolated and purified by chromatography.

### PREPARATION OF THE SALT OF FORMULA I

The pharmaceutically acceptable salts of Formula I are prepared by dissolving a compound of Formula I in a suitable solvent (such as methanol) adding 1 to 3 molar equivalents (preferably about two molar equivalent) of an appropriate acid (such as hydrochloric acid) or base (such as an alkaline earth hydroxide, e.g., lithium hydroxide, calcium hydroxide, potassium hydroxide, sodium hydroxide, preferably sodium hydroxide) with stirring. The salt is isolated by lyophilization or by precipitation, using techniques that will be apparent to those skilled in the art.

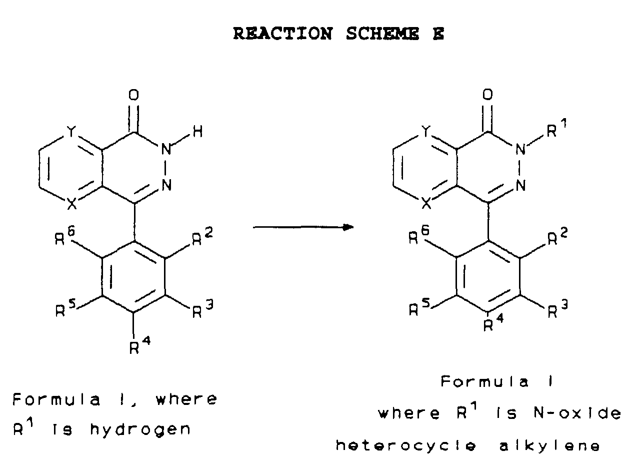

### PREPARATION OF FORMULA I WHERE R¹ IS PYRIDYL-N-OXIDE-ALKYL.

Optionally substituted pyridyl-N-oxide-alkyl pyridopyridazinones are prepared by following Mitsunobu reaction conditions, as illustrated in O. Mitsunobu, *Synthesis,* 1, 1981, and incorporated herein by reference. An optionally substituted pyridopyridazinone (Formula I) where R¹ is hydrogen is combined into a suspension with about 1.1 molar equivalents of an optionally substituted pyridylcarbinol N-oxide, about 1.5 molar equivalents of triphenylphosphine in a solvent (e.g., tetrahydrofuran, diethyl ether, dioxane, preferably tetrahydrofuran). The suspension is stirred for a period of about 12 to 24 hours, preferably about 18 hours, at a temperature in the range of 12 to 36°C, preferable about 24°C. The solvent is removed and the desired optionally substituted pyridyl-N-oxide-alkyl pyridopyridazinone is isolated by chromatography, e.g., elution by 100% ethylacetate, followed by 10% methanol/ethylacetate or like solvent systems.

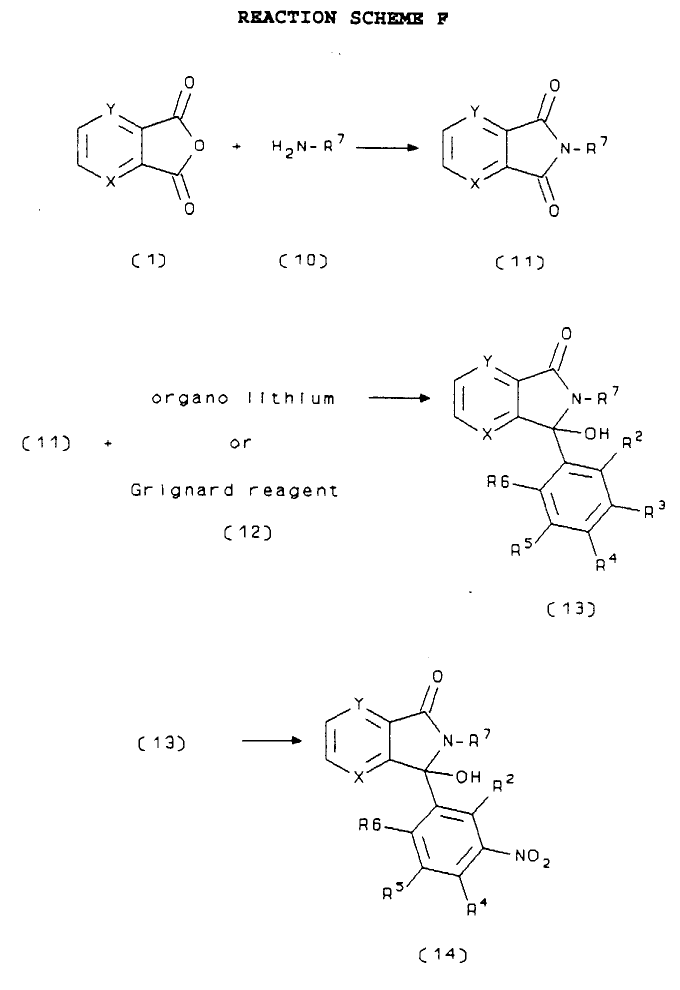

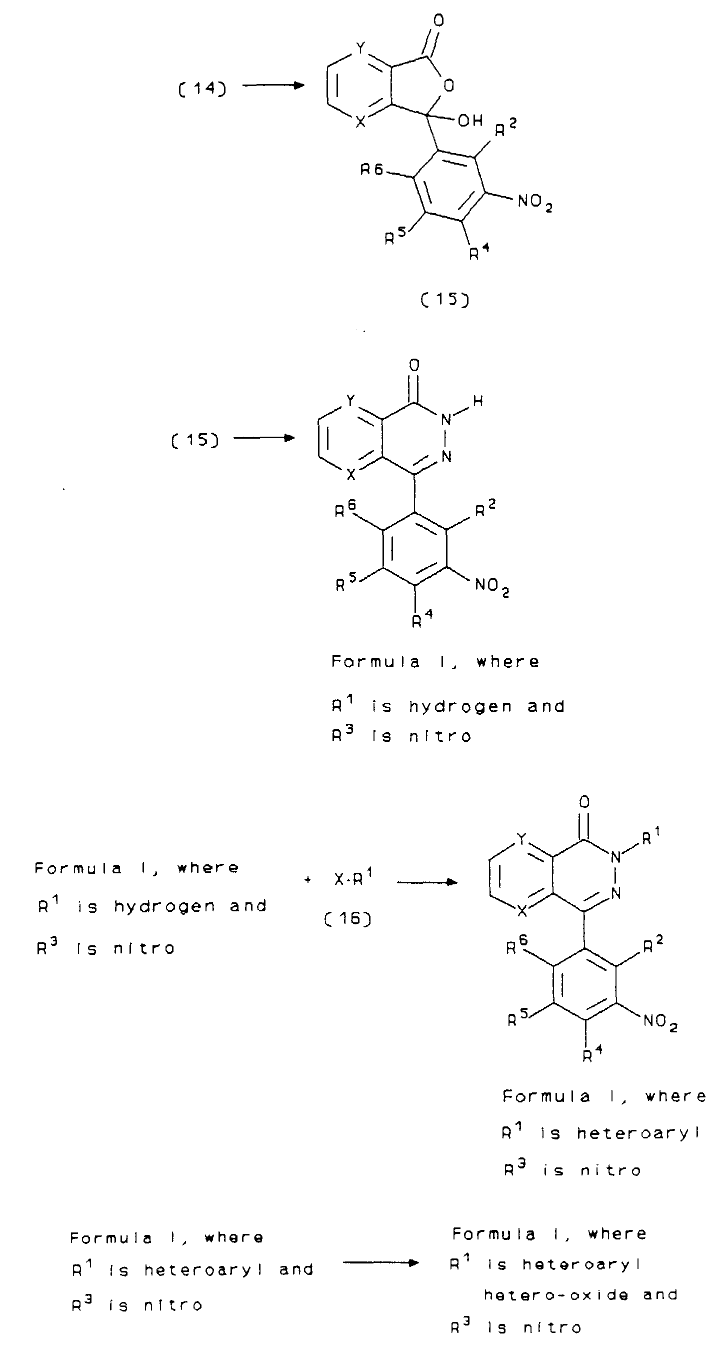

### PREPARATION OF A COMPOUND OF FORMULA 11

A pyridodicarboxylic anhydride (Formula 1, where X is nitrogen and Y is carbon) is suspended in a solvent (such as toluene, ethyl acetate, tetrahydrofuran, preferably ethyl acetate) and a primary amine (Formula 10, where R⁷ is lower alkyl, such as, ethyl, propyl, butyl, hexyl, or n-butyl, preferably n-butyl) is added. The temperature is kept in a range of about -10°C to about 80°C, preferably room temperature. After a period of about 10 minutes to about 24 hours, preferably about 1 hour, a dehydrating reagent (such as, acetic anhydride, trifluoroacetic anhydride, phosphorous pentoxide, or thionyl chloride, preferably thionyl chloride) is added. The temperature of the mixture is kept in a range of about 0°C to about 80°C (depending on the dehydrating reagent) for a period of about 30 minutes to about 4 hours, preferably about 1 hour. The resulting pyridoimide (Formula 11, where X is nitrogen, Y is carbon and R⁷ is lower alkyl) is isolated by chromatography or crystallization, preferably crystallization.

### PREPARATION OF A COMPOUND OF FORMULA 13

The pyridoimide (Formula 11) is dissolved in a solvent that is inert to Grignard reagents and organolithium reagents (e.g., tetrahydrofuran, diethyl ether, toluene, preferably toluene) and cooled to a temperature in the range of about -100°C to room temperature, preferably about -65°C. About 0.5 to 3.0 molar equivalent, preferably about 1.0 molar equivalent of an optionally substituted phenyl Grignard reagent or optionally substituted phenyl organolithium reagent, preferably an optionally substituted phenyl Grignard reagent (Formula 8) in either tetrahydrofuran or diethyl ether, preferably tetrahydrofuran is added at such a rate that the internal temperature of the solution is maintained in a range of about -78°C to about room temperature, preferably about -45°C. After a period of about 10 minutes to about 5 hours, preferably about 30 minutes, the reaction is quenched with dilute hydrochloric acid, dilute acetic acid, aqueous ammonium chloride solution, dilute sulfuric acid, or the like, preferably aqueous ammonium chloride solution. The resulting phenyl pyridolactam compound (Formula 13 where X is nitrogen, Y is carbon, R⁷ is lower alkyl, and R³ is hydrogen) can be isolated by chromatography, crystallization or used as a crude mixture in the next reaction, preferably the product is isolated by crystallization.

### PREPARATION OF A COMPOUND OF FORMULA 14 WHERE R³ IS NITRO

The phenyl pyridolactam compound (Formula 13) is suspended in sulfuric acid and cooled to a temperature in the range of about -10°C to about 40°C, preferably room temperature. Nitric acid is added at such a rate that the temperature is maintained below about 60°C, preferably below about 45°C. Following the addition of the nitric acid the mixture is stirred for a period of about 10 minutes to about 6 hours, preferably about 30 minutes. The mixture is then added to water and extracted with a water immiscible solvent (e.g., diethyl ether, ethyl acetate, toluene, methylene chloride, preferably ethyl acetate). The resulting optionally substituted nitrophenyl pyridolactam compound (Formula 14 where X is nitrogen, Y is carbon, and R³ is nitro) can be isolated by chromatography, crystallization or preferably used directly in the next step.

### PREPARATION OF A COMPOUND OF FORMULA 15 WHERE R³ IS NITRO

An optionally substituted nitrophenyl pyridolactam compound (Formula 14) as prepared in the previous step is dissolved or suspended in about 2 to 6 molar equivalent, preferably about 3 molar equivalent, dilute acid (such as, hydrochloric acid, hydrobromic acid, sulfuric acid, preferably hydrochloric acid) and heated to reflux for about 4 hours to about 48 hours, preferably about 24 hours.
The optionally substituted nitrophenyl pyridolactone compound (Formula 15 where X is nitrogen, Y is carbon, and R³ is nitro) is isolated by filtration or neutralization and extraction, followed by crystallization or chromatography, preferably filtration.

### PREPARATION OF A COMPOUND OF FORMULA I, WHERE R¹ IS HYDROGEN AND R³ IS NITRO

An optionally substituted nitrophenyl pyridolactone compound (Formula 15) is mixed with a solvent inert and miscible with hydrazine (e.g., methanol, ethanol, dimethylformamide, preferably methanol) and heated to a temperature in the range of about room temperature to about 80°C, preferably about 65°C. After stirring the reaction for a period of about 1 hour to about 24 hours, preferably about 18 hours, an optionally substituted nitrophenyl pyridopyridazinone (Formula I where X is nitrogen, Y is carbon, Z is oxygen, R¹ is hydrogen, and R³ is nitro) is isolated by filtration.

### PREPARATION OF A COMPOUND OF FORMULA I WHERE R¹ IS HETEROARYL AND R³ IS NITRO

An optionally substituted nitrophenyl pyridopyridazinone (Formula I where X is nitrogen, Y is carbon, R¹ is hydrogen and R³ is nitro) is dissolved in an aprotic solvent (e.g., tetrahydrofuran, dimethylformamide, dimethylacetamide, preferably dimethylformamide) and treated with a base (e.g., sodium hydride, potassium hydride, preferably sodium hydride). The resulting solution is heated to a temperature in the range of about room temperature to about 95°C, preferably about 65°C. About 1.2 molar equivalent of an optionally substituted heteroaryl alkylating reagent (Formula 16, where R¹ is heteroaryl) is added. The resulting optionally substituted nitrophenyl heteroaryl pyridopyridazinone (Formula I where X is nitrogen, Y is carbon, Z is oxygen, R¹ is heteroaryl, and R³ is nitro) is isolated by crystallization or chromatography, preferably crystallization.

### PREPARATION OF A COMPOUND OF FORMULA I WHERE R¹ IS HETEROARYL HETERO-OXIDE AND R³ IS NITRO

An optionally substituted nitrophenyl heteroaryl pyridopyridazinone (Formula I where X is nitrogen, Y is carbon, R¹ is heteroaryl and R³ is nitro) is dissolved in a solvent compatible with peroxyacid (e.g., ethyl acetate, toluene, methylene chloride, acetic acid, preferably methylene chloride) and a peroxyacid (such as, m-chloroperoxybenzoic acid, peroxyacetic acid, trifluoroperoxyacetic acid, magnesium monoperoxyphthalate, preferably m-chloroperoxybenzoic acid) is added. The resulting mixture is stirred for a period of about 1 hour to about 48 hours, preferably about 18 hours at a temperature in the range of about 0°C to about 50°C, preferably room temperature. The resulting optionally substituted nitrophenyl heteroaryl hetero-oxide pyridopyridazinone (Formula I, where X is nitrogen, Y is carbon, Z is oxygen, R¹ is heteroaryl hetero-oxide and R³ is nitro) is isolated by crystallization or chromatography, preferably crystallization.

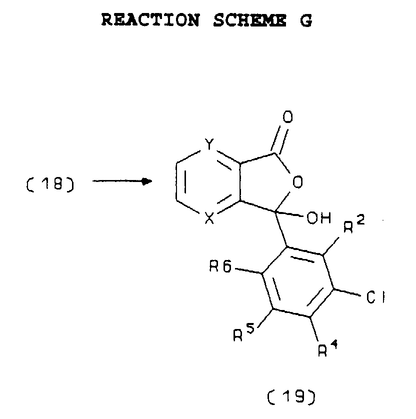

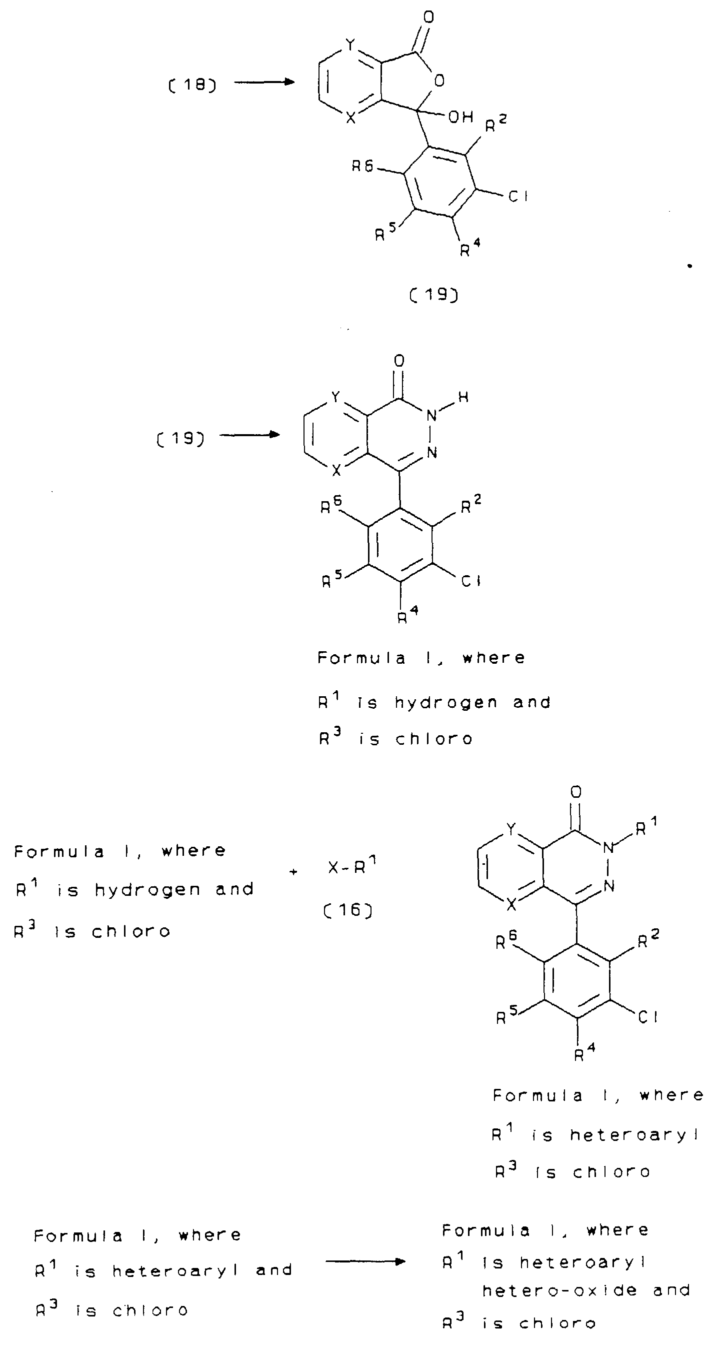

### PREPARATION OF A COMPOUND OF FORMULA 18 WHERE R³ IS CHLORO

An optionally substituted m-chlorophenyl pyridolactam is made by following the procedure for preparing Formula 13 set forth in Reaction Scheme F. An optionally substituted *m*-chloro Grignard reagent or organolithium reagent (Formula 17) is used in lieu of Formula 12, resulting in the desired optionally substituted *m*-chlorophenyl pyridolactam (Formula 18, where X is nitrogen, Y is carbon and R³ is chloro).

### PREPARATION OF A COMPOUND OF FORMULA 19 WHERE R³ IS CHLORO

An optionally substituted *m*-chlorophenyl pyridolactone (Formula 19 where R³ is chloro) is prepared by following the procedure for preparing Formula 15 set forth in Reaction Scheme F.

### PREPARATION OF COMPOUND OF FORMULA I WHERE R¹ IS HYDROGEN AND R³ IS CHLORO

An optionally substituted *m*-chlorophenyl pyridopyridazinone (Formula I where R¹ is hydrogen and R³ is chloro) is prepared by following the procedure for preparing Formula I where R¹ is hydrogen and R³ is nitro set forth in Reaction Scheme F.

### PREPARATION OF A COMPOUND OF FORMULA I WHERE R¹ IS HETEROARYL AND R³ IS CHLORO

An optionally substituted *m*-chlorophenyl heteroaryl pyridopyridazinone (Formula I where R¹ is heteroaryl and R³ is chloro) is prepared by following the procedure for preparing Formula I where R¹ is heteroaryl and R³ is nitro set forth in Reaction Scheme F.

### PREPARATION OF A COMPOUND OF FORMULA I WHERE R¹ IS HETEROARYL HETERO-OXIDE AND R³ IS CHLORO

An optionally substituted *m*-chlorophenyl heteroaryl hetero-oxide pyridopyridazinone (Formula I where R¹ is heteroaryl and R³ is chloro) is prepared by following the procedure for preparing Formula I where R¹ is heteroaryl hetero-oxide and R³ is nitro set forth in Reaction Scheme F.

### PREFERRED COMPOUNDS

Presently preferred are the compound of Formula I where X is nitrogen, Y is carbon, Z is oxygen, R³ is chloro or nitro and R¹ is ethyl, 2-propyl, 4-pyridylmethyl, 4-pyridyl-N-oxide-methyl, 3-pyridylmethyl, 3-pyridyl-N-oxide-methyl, benzyl, 3-thienylmethyl or cyclopentylmethyl.

Preferred are the compounds of Formula I where R³ is chloro and R¹ is ethyl, 4-pyridylmethyl, 4-pyridyl-N-oxide-methyl, 3-pyridylmethyl, 3-pyridyl-N-oxide-methyl, benzyl or 3-thienylmethyl;
particularly the compounds where R², R⁴, R⁵ and R⁶ are hydrogen.

Similarly preferred are the compounds of Formula I where R³ is nitro and R¹ is 4-pyridylmethyl, 4-pyridyl-N-oxide-methyl, 3-pyridylmethyl, 3-pyridyl-N-oxide-methyl, benzyl, 3-thienylmethyl, 2-propyl or cyclopentyl;
particularly the compounds where R², R⁴, R⁵ and R⁶ are hydrogen.

Most preferred are the compounds 6-(4-pyridylmethyl)-8-(3-nitrophenyl)-pyrido[2,3-d]pyridazin-5-one, 6-(4-pyridyl-N-oxidemethyl)-8-(3-nitrophenyl)pyrido[2,3-d]pyridazin-5-one, 6-(3-pyridylmethyl)-8-(3-nitrophenyl)pyrido[2,3-d]pyridazin-5-one, 6-(3-pyridyl-N-oxide-methyl)-8-(3-nitrophenyl)pyrido[2,3-d]pyridazin-5-one, 6-benzyl-8-(3-nitrophenyl)pyrido[2,3-d]pyridazin-5-one, 6-ethyl-8-(3-nitrophenyl)pyrido[2,3-d]pyridazin-5-one and 6-(3-thienylmethyl)-8-(3-nitrophenyl)pyrido[2,3-d]pyridazin-5-one.

Also most preferred are the compounds 6-(4-pyridylmethyl)-8-(3-chlorophenyl)-pyrido[2,3-d]pyridazin-5-one, 6-(4-pyridyl-N-oxidemethyl)-8-(3-chlorophenyl)pyrido[2,3-d]pyridazin-5-one, 6-(3-pyridylmethyl)-8-(3-chlorophenyl)pyrido[2,3-d]pyridazin-5-one, 6-(3-pyridylmethyl)-8-(3-chlorophenyl)pyrido[2,3-d]pyridazin-5-one, 6-benzyl-8-(3-chlorophenyl)pyrido[2,3-d]pyridazin-5-one, 6-(2-propyl)-8-(3-chlorophenyl)pyrido[2,3-d]pyridazin-5-one, 6-cyclopentyl-8-(3-chlorophenyl)pyrido[2,3-d]pyridazin-5-one, and 6-(3-thienylmethyl)-8-(3-chlorophenyl)pyrido[2,3-d]pyridazin-5-one.

### PREFERRED PROCESSES AND LAST STEPS

A preferred process for making optionally substituted pyrido[2,3-d]pyridazin-5-one or pyrido[2,3-d]pyridazin-8-one compounds entails contacting a suitably substituted 2-benzoyl-3-methoxycarbonylpyridine with a suitably substituted hydrazine.

A preferred process for making optionally substituted 2-benzoyl-3-methoxycarbonylpyridine entails contacting a suitably substituted 2-benzoyl-3-carboxypyridine with an esterification reagent.

A preferred process for making optionally substituted 2-benzoyl-3-carboxypyridine entails contacting a suitably substituted pyridodicarboxylic anhydride with a suitably substituted Grignard reagent.

A preferred process for making 6-substituted pyrido[2,3-d]pyridazin-5-one entails contacting of the 6-unsubstituted pyrido[2,3-d]pyridazin-5-one with a suitable base, followed by the addition of an alkylating agent.

A preferred process for making 7-substituted pyrido[2,3-d]pyridazin-8-one entails contacting of the 7-unsubstituted pyrido[2,3-d]pyridazin-8-one with a suitable base, followed by the addition of an alkylating agent.

A preferred process for making 6-substituted pyrido[2,3-d]pyridazin-5-one entails contacting the 6-unsubstituted pyrido[2,3-d]pyridazin-5-one compound with a suitably substituted alcohol and triphenyl phosphine, followed by diisopropyl azodicarboxylate (i.e., Mitsunobu reaction conditions).

A preferred process for making 7-substituted pyrido[2,3-d]pyridazin-8-one entails contacting the 7-unsubstituted pyrido[2,3-d]pyridazin-8-one compound with a suitably substituted alcohol and triphenyl phosphine, followed by diisopropyl azodicarboxylate (i.e., Mitsunobu reaction conditions).

A preferred process for making 6-substituted pyrido-N-oxidealkyl[2,3-d]pyridazin-5-one entails suspending the 6-unsubstituted pyrido[2,3-d]pyridazin-5-one compound with a suitably substituted pyridylcarbinol N-oxide and triphenylphosphine, followed by diisopropyl azodicarboxylate (i.e., Mitsunobu reaction conditions).

A preferred process for making 7-substituted pyrido-N-oxidealkyl[2,3-d]pyridazin-8-one entails suspending the 7-unsubstituted pyrido[2,3-d]pyridazin-8-one compound with a suitably substituted pyridylcarbinol N-oxide and triphenylphosphine, followed by diisopropyl azodicarboxylate (i.e., Mitsunobu reaction conditions).

A preferred process for converting a 6,8-disubstituted pyrido[2,3-d]pyridazin-5-one into the corresponding 6,8-disubstituted pyrido[2,3-d]pyridazin-5-thione entails treating the 6,8-disubstituted pyrido[2,3-d]pyridazin-5-one with a reagent for the thiation of ketones (such as Lawesson's Reagent or phosphorus pentasulfide) in an appropriate solvent, preferably at reflux.

### UTILITY, TESTING AND ADMINISTRATION

### GENERAL UTILITY

The compounds of this invention, including the pharmaceutically acceptable salts and esters thereof, and the compositions containing them are particularly useful as anti-inflammatory, antasthmatc, immunosuppressive, anti-allograft rejection, anti-graft-vs-host disease, autoimmune disease or analgetic agents. The compounds of this invention act as PDE IV selective inhibitors, thereby modulating CAMP levels. Thus, these compounds are of use for the treatment of conditions or diseases that are modulated by leukocyte cAMP.

For example, inflammation, immunosuppression, auto-immune diseases, graft-vs-host disease and allograft rejection are conditions that are manifested by the proliferation of lymphocytes.
The proliferation is triggered by the presence of cAMP at specific levels. Inhibition of lymphocyte proliferation is accomplished by increasing levels of cAMP resulting from the inhibition of lymphocyte phosphodiesterase.

### TESTING

Potency and selectivity of compounds as inhibitors of PDE IV is determined by following, for example, the procedures described in Example 46, or modifications thereof.

The immunomodulatory and anti-inflammatory activity of the compounds of the invention can be determined by a variety of assays utilizing both *in vitro* and *in vivo* procedures.

Inhibition of the proliferation of lymphocytes in response to mitogenic stimulation is determined by the procedures described by Greaves, et al. ["Activation of human T and B lymphocytes by polyclonal mitogens," *Nature,* 248, 698-701 (1974)] ., or modifications thereof (see Example 48).

Inhibition of lymphocyte activation in response to antigenic challenge is determined *in vitro* by inhibition of a cytolytic T-cell assay (CTL) as described by Wunderlich, et al., *Nature* (1970), Vol. 228, p. 62, or a modification thereof.

Immune modulation is determined by *in vivo* procedures utilizing the Jerne Hemolytic Plaque Assay, [Jerne, et al., "The agar plaque technique for recognizing antibody producing cells," *Cell-bound Antibodies,* Amos, B. and Kaprowski, H. editors (Wistar Institute Press, Philadelphia) 1963, p. 109] or a modification thereof (see Example 47).

Anti-inflammatory activity is determined by the Arachidonic Acid-Induced Mouse Ear Edema Assay [Young, et al., *J. Invest. Derm., 82:* 367-371 (1984)] (see Example 49).

Analgetic activity is determined by the Phenylquinone-induced Mouse Writhing Assay [Hendershot, et al., *J. Pharmacol. Exp. Ther., 125:* 237-240 (1959)].

### ADMINISTRATION

The compounds of this invention are administered at a therapeutically effective dosage, i.e., that amount which, when administered to a mammal in need thereof, is sufficient to effect treatment, as described above (for example, to reduce or otherwise treat inflammation, pain and/or pyrexia in the mammal). Administration of the active compounds and salts described herein can be via any of the accepted modes of administration for agents that serve similar utilities.

The level of the drug in a formulation can vary within the full range employed by those skilled in the art, e.g., from about 0.01 percent weight (%w) to about 99.99%w of the drug based on the total formulation and about .01%w to 99.99%w excipient. Preferably the drug is present at a level of about 10%w to about 70%w.

Generally, an acceptable daily dose is of about 0.0001 to 150 mg per kilogram body weight of the recipient per day, preferably about 0.01 to 75 mg per kilogram body weight per day, and most preferably about 0.1 to 30 mg per kilogram body weight per day. Thus, for administration to a 70 kg person, the dosage range would be about 0.007 mg to 10.5 g per day, preferably about 0.7 to 5.25 g per day, and most preferably about 7.0 mg to 2.1 g per day.

Administration can be via any accepted systemic or local route, for example, via parenteral, oral (particularly for infant formulations), intravenous, nasal, transdermal or topical routes, in the form of solid, semi-solid or liquid dosage forms, such as for example, tablets, suppositories, pills, capsules, powders, solutions, suspensions, aerosols, emulsions or the like, preferably in unit dosage forms suitable for simple administration of precise dosages. The compositions will include a conventional pharmaceutical carrier or excipient and an active compound of Formula I and, in addition, may include other medicinal agents, pharmaceutical agents, carriers, adjuvants, etc. Carriers can be selected from the various oils, including those of petroleum, animal, vegetable or synthetic origin, for example, peanut oil, soybean oil, mineral oil, sesame oil, and the like. Water, saline, aqueous dextrose, and glycols are preferred liquid carriers, particularly for injectable solutions. Suitable pharmaceutical carriers include starch, cellulose, talc, glucose, lactose, sucrose, gelatin, malt, rice, flour, chalk, silica gel, magnesium stearate, sodium stearate, glycerol monostearate, sodium chloride, dried skim milk, glycerol, propylene glycol, water, ethanol, and the like. Other suitable pharmaceutical carriers and their formulations are described in "*Remington's Pharmaceutical Sciences*" by E. W. Martin.

If desired, the pharmaceutical composition to be administered may also contain minor amounts of non-toxic auxiliary substances such as wetting or emulsifying agents, pH buffering agents and the like, such as for example, sodium acetate, sorbitan monolaurate, triethanolamine oleate, etc.

The compounds of this invention are generally administered as a pharmaceutical composition which comprises a pharmaceutical excipient in combination with a compound of Formula I. The level of the drug in a formulation can vary within the full range employed by those skilled in the art, e.g., from about .01 percent weight (%w) to about 99.99%w of the drug based on the total formulation and about .01%w to 99.99%w excipient. Preferably, the formulation will be about 3.5 to 60% by weight of the pharmaceutically active compound, with the rest being suitable pharmaceutical excipients.

### INTRAVENOUS ADMINISTRATION

Intravenous injection has proven to be an important route of administration for antiviral agents. The compounds of the present invention can be administered via this route, for example, by dissolving the compound, salt, ester or ether in a suitable solvent (such as water or saline) or incorporation in a liposomal formulation followed, by dispersal into an acceptable infusion fluid. A typical daily dose of a compound of the invention can be administered by one infusion, or by a series of infusions spaced over periodic intervals.

### ORAL ADMINISTRATION

Oral administration can be used to deliver the compound of Formula I using a convenient daily dosage regimen which can be adjusted according to the degree of affliction or for renal impairment, or to compensate for the toxic effects of other medications administered contemporaneously. For such oral administration, a pharmaceutically acceptable, non-toxic composition is formed by the incorporation of any of the normally employed excipients, such as, for example, pharmaceutical grades of mannitol, lactose, starch, magnesium stearate, sodium saccharine, talcum, cellulose, glucose, gelatin, sucrose, magnesium carbonate, and the like. Such compositions take the form of solutions, suspensions, tablets, pills, capsules, powders, sustained release formulations and the like. Such compositions may contain between .01 wt/wt% and 99.99 wt/wt% of the compound of Formula I, but preferably such compositions will contain between 25 wt/wt% and about 80 wt/wt%.

Preferably the compositions will take the form of a capsule, pill or tablet and thus the composition will contain, along with the active ingredient, a diluent such as lactose, sucrose, dicalcium phosphate, and the like; a disintegrant such as starch or derivatives thereof; a lubricant such as magnesium stearate and the like; and a binder such as a starch, polyvinylpyrrolidone, gum acacia, gelatin, cellulose and derivatives thereof, and the like. For oral administration to infants, a liquid formulation (such as a syrup or suspension) is preferred.

### LIPOSOMAL FORMULATIONS

Pharmaceutical formulations based on liposomes have recently reached human clinical trials. Their benefits are believed related to favorable changes in tissue distribution and pharmacokinetic parameters that result from liposome entrapment of drugs, and may be applied to the compounds of the present invention by those skilled in the art.

The formulations can be designed to either target drug to disease sites [see: Lopez-Berestein et al., *J. Infect. Dis.*, *151*: 704-710 (1985); Gotfredsen et al., *Biochemical Pharmacology, 32*: 3389-3396 (1983)]; or to the reticuloendothelial system [see Eppstein et al., *Int. J. Immunotherapy, 2:* 115-126 (1986)1, to increase duration of drug action [see: Gabizon et al., *Cancer Res., 42:* 4734 (1982); Eppstein et al., *Delivery Systems for Peptide Drugs,* Eds. S.S. Davis, L. Illum and E. Tomlinson, Plenum Pub. Corp., New York, pp. 277-283; C.A. Hunt, *Biochemica* et *Biophysica Acta., 719*: 450-463 (1982); and Senior et al., *Biochemica et Biophysica Acta.*, *839:* 1-8 (1985)], or to divert a drug away from organs that are particularly sensitive to its toxic effects [see: Weinstein et al., *Pharmac. Ther., 24*: 207-233 (1983); Olson et al., *Eur. J. Cancer Clin. Oncol., 18*: 167-176 (1982); and Gabzion et al., *supra.*]*.*

Controlled release liposomal liquid pharmaceutical formulations for injection or oral administration are described in U.S. Patent No. 4,016,100. Liposomal applications for oral drug delivery of a lyophilized liposome/peptide drug mixture filled into intestine capsules have also been suggested, see U.S. Patent No. 4,348,384. The foregoing is incorporated herein by reference.

### SUPPOSITORIES

For systemic administration via suppository, traditional binders and carriers include, for example, polyalkaline glycol or triglycerides [e.g., PEG 1000 (96%) and PEG 4000 (4%)]. Such suppositories may be formed from mixtures containing active ingredients in the range of from about 0.5 wt/wt% to about 10 wt/wt%; preferably from about 1 wt/wt% to about 2 wt/wt%.

### LIQUIDS

Liquid pharmaceutically administrable compositions can, for example, be prepared by dissolving, dispersing, etc. an active compound (about 0.5% to about 20%), as described above, and optional pharmaceutical adjuvants in a carrier, such as, for example, water, saline, aqueous dextrose, glycerol, ethanol and the like, to thereby form a solution or suspension.

Actual methods of preparing such dosage forms are known, or will be apparent, to those skilled in this art; for example, see *Remington's Pharmaceutical Sciences*, Mack Publishing Company, Easton, Pennsylvania, 16th Ed., 1980. The composition to be administered will, in any event, contain a quantity of the active compound(s) in a pharmaceutically effective amount for relief of the particular condition being treated in accordance with the teachings of this invention.

### EXAMPLES

The following preparations and examples are given to enable those skilled in the art to more clearly understand and to practice the present invention. They should not be considered as limiting the scope of the invention, but merely as being illustrative and representative thereof.

### EXAMPLE 1

### Preparation of 7-(3-nitrophenyl)-furo[3,4-b]pyridin-5-one

### 1. Formula 4, Where X is nitrogen, Y is carbon, R³ is nitro, and R², R⁴, R⁵ and R⁶ are hydrogen.

A solution of 2,3-pyridinyldicarboxylic anhydride (20g, 134mmoles) in 25ml of anhydrous methanol was refluxed for 30 minutes. The methanol was removed and the two positional isomers (2-carboxy-3-methoxycarbonylpyridine and 2-methoxycarbonyl-3-carboxypyridine) are isolated and separated. The 2-carboxy-3-methoxycarbonylpyridine was added to a solution of 3-nitrobenzaldehyde (101.35g, 670mmoles) in p-cymene (105ml, 670mmoles) and heated at 180°C for 13.5 hours. The solution was cooled, filtered and the excess 3-nitrobenzaldehyde and p-cymene removed by bulb to bulb distillation using a Kugelrohr apparatus. The residue was triturated with methylene chloride and the solid residue was discarded. The product was isolated from the filtrate yielding 12.7g of 7-(3-nitrophenyl)furo[3,4-b]pyridin-5-one (37.2%).

### EXAMPLE 2

### Preparation of 2-(hydroxymethyl-3-nitrophenyl)-3-carboxypyridine

### 2. Formula 5, Where X is nitrogen, Y is carbon, R³ is nitro and R², R⁴, R⁵ and R⁶ are hydrogen.

7-(3-nitrophenyl)-furo[3,4-b]pyridin-5-one was suspended in methanol (100ml), solid potassium hydroxide (4.5g, 80mmoles) was added and the mixture refluxed for 6 hours under an inert atmosphere yielding 2-(hydroxymethyl-3-nitrophenyl)-3-carboxypyridine.

### Example 3

### Preparation of 2-(3-nitrobenzoyl)-3-carboxypyridine

### 3. Formula 6, Where X is nitrogen, Y is carbon, R³ is nitro and R², R⁴, R⁵ and R⁶ are hydrogen.

To a solution of 2-(hydroxymethyl-3-nitrophenyl)-3-carboxypyridine (2.25g, 8.2mmoles) in methylene chloride (20ml), pyridinium dichromate (4.63g, 12.3mmoles) was added and stirred at room temperature (22°C) for 18 hours. The solution was filtered and the solvent removed yielding 3.43g of crude 2-(3-nitrobenzoyl)-3-carboxypyridine.

### Example 4

### Preparation of 2-(3-nitrobenzoyl)-3-methoxycarbonylpyridine

### 4. Formula II, Where X is nitrogen, Y is carbon, R³ is nitro and R², R⁴, R⁵ and R⁶ are hydrogen.

2-(3-Nitrobenzoyl)-3-carboxypyridine was dissolved in 20ml of methylene chloride and the solution was cooled in an ice/water bath. Diazomethane (25ml, 0.33M) was added to the solution and stirred for 2 hours. The solvent was removed and the residue chromatographed (methylene chloride) yielding 0.26g of 2-(3-nitrobenzoyl)-3-methoxycarbonylpyridine.

### EXAMPLE 5

### Preparation of 8-(3-nitrophenyl)pyrido[2,3-d]pyridazin-5-one

### 5. Formula I, Where X is nitrogen, Y is carbon, Z is oxygen, R³ is nitro and R¹, R², R⁴, R⁵ and R⁶ are hydrogen.

To a solution of 2-(3-nitrobenzoyl)-3-methoxycarbonylpyridine (0.58g, 2mmoles) dissolved in ethanol (50ml), was added hydrazine monohydrate (0.25ml, 5.1mmoles). The mixture was refluxed for 4 hours, cooled and ethyl acetate (50ml) was added. The solid material was collected and dried, yielding 0.58g of 8-(3-nitrophenyl)pyrido[2,3-d]pyridazin-5-one, mp. 302°C.

### EXAMPLE 6

### Preparation of 6-phenyl-8-(3-nitrophenyl)pyrido[2,3-d]pyridazin-5-one

### 6. Formula I, Where X is nitrogen, Y is carbon, Z is oxygen, R¹ is phenyl, R³ is nitro and R², R⁴, R⁵ and R⁶ are hydrogen.

To a solution of 2-(3-nitrobenzoyl)-3-methoxycarbonylpyridine (0.26g, 0.97mmoles) dissolved in ethanol (50ml), was added phenylhydrazine (0.25ml, 2.4mmoles). The mixture was refluxed for 168 hours. The solvent was removed and the solid was precipitated from ethanol and dried, yielding 0.07g of 6-phenyl-8-(3-nitrophenyl)pyrido[2,3-d]pyridazin-5-one (21%), mp 167°C.

### EXAMPLE 7

### Preparation of 6-benzyl-8-(3-nitrophenyl)pyrido-[2,3-d]pyridazin-5-one

### 7. Formula I, Where X is nitrogen, Y is carbon, Z is oxygen, R¹ is benzyl, R³ is nitro and R², R⁴, R⁵ and R⁶ are hydrogen.

To a solution of 2-(3-nitrobenzoyl)-3-methoxycarbonylpyridine (0.20g, 0.75mmoles) dissolved in ethanol (50ml), was added benzylhydrazine hydrochloride (0.36g, 1.86mmoles). The mixture was heated to 85°C for 144 hours. The mixture was purified by chromatography yielding 0.llg of 6-benzyl-8-(3-nitrophenyl)pyrido[2,3-d]pyridazin-5-one (41%), mp 132°C.

### EXAMPLE 8

### Preparation of 6-ethyl-8-(3-nitrophenyl)pyrido[2,3-d]pyridazin-5-one

### 8. Formula I, Where X is nitrogen, Y is carbon, Z is oxygen, R¹ is ethyl, R³ is nitro and R², R⁴, R⁵ and R⁶ are hydrogen.

8-(3-Nitrophenyl)-pyrido[2,3-d]pyridazin-5-one (0.20g, 0.75mmoles), sodium hydride (0.04g, 0.9mmoles) and iodoethane (0.3ml, 3.75mmoles) was dissolved in 25ml of tetrahydrofuran and refluxed under a nitrogen atmosphere for 18 hours. The solvent was removed and the product chromatographed and eluted with 100% methylene chloride yielding 0.05g of 6-ethyl-8-(3-nitrophenyl)pyrido[2,3-d]pyridazin-5-one (22.6%), mp. 138°C.

### EXAMPLE 9

### Preparation of 6-(4-pyridylmethyl)-8-(3-nitrophenyl)pyrido[2,3-d]pyridazin-5-one

### 9. Formula I, Where X is nitrogen, Y is carbon, Z is oxygen, R¹ is 4-pyridylmethyl, R³ is nitro and R², R⁴, R⁵ and R⁶ are hydrogen.

To a solution of 8-(3-nitrophenyl)-pyrido[2,3-d]pyridazin-5-one (0.16 g, 0.6 mmoles) in tetrahydrofuran (50ml) was added potassium carbonate (0.19 g, 7.1 mmoles), potassium iodide (1.0 g, 6.0 mmoles) and 4-picolyl chloride (0.98 g, 6.0 mmoles). The mixture was stirred and refluxed under an inert atmosphere for 18 hours. The solvent was removed under vacuum. The residue was partitioned in methanol/ethyl acetate (1:1), chromatographed in 100% hexane followed by 100% ethyl acetate. The remaining solvent was removed yielding 0.58g of crude 6-(4-pyridylmethyl)-8-(3-nitrophenyl)pyrido[2,3-d]pyridazin-5-one, which was recrystallized from methanol yielding 0.08 g of the pure compound (37.1%), mp. 167-168°C.

### EXAMPLE 10

### Preparation of 7-(3-chlorophenyl)-furo[3,4-b]pyridin-5-one

### 10. Formula 4, Where X is nitrogen, Y is carbon, R³ is chloro and R², R⁴, R⁵ and R⁶ are hydrogen.

A solution of 2,3-pyridinyldicarboxylic anhydride (20.0g, 134mmoles) in methanol (25g, 617mmoles) was refluxed for 10 minutes and the solvent removed. The solution was filtered and the remaining solvent was removed. The residue was added to 3-chlorobenzaldehyde (76 ml, 671 mmoles) and p-cymene (105 ml, 671 mmoles) and the mixture was stirred at 180°C for 18 hours under an inert atmosphere. The solvent was removed and the residue was chromatographed yielding 7-(3-chlorophenyl)furo[3,4-b]pyridin-5-one.

### EXAMPLE 11

### Preparation of 2-(hydroxymethyl-3-chlorophenyl)-3-carboxypyridine

### 11. Formula 5, Where X is nitrogen, Y is carbon, R³ is chloro and R², R⁴, R⁵ and R⁶ are hydrogen.

7-(3-Chlorobenzoyl)furo[3,4-b]pyridin-5-one (4.77g, 19.4mmoles) and 0.2N sodium hydroxide (21.4mmoles) was dissolved in methanol and tetrahydrofuran (100ml, 1:1). The mixture was heated to 75°C and stirred for 3 hours and cooled. 1N Hydrochloric acid (20ml) and water (200ml) was added to the mixture. The resulting solution was extracted with ethyl acetate and dried over Na₂SO₄. The solution was filtered and the solvent removed yielding 4.94g (96.8%) of 2-(hydroxymethyl-3-chlorophenyl)-3-carboxypyridine.

### EXAMPLE 12

### Preparation of 2-(3-chlorobenzoyl)-3-carboxypyridine

### 12. Formula 6, Where X is nitrogen, Y is carbon, R³ is chloro and R², R⁴, R⁵ and R⁶ are hydrogen.

2-(Hydroxymethyl-3-chlorophenyl)-3-carboxypyridine was dissolved in a solution of methylene chloride/glacial acetic acid (5:1). Pyridinium dichromate (10.95g, 29mmoles) was added and the solution was stirred at room temperature under an inert atmosphere. The solvent was removed yielding 2-(3-chlorobenzoyl)-3-carboxypyridine.

### EXAMPLE 13

### Preparation of 2-(3-chlorobenzoyl)-3-methoxycarbonylpyridine

### 13. Formula II, Where X is nitrogen, Y is carbon, R³ is chloro and R², R⁴, R⁵ and R⁶ are hydrogen.

2-(3-Chlorobenzoyl)-3-carboxypyridine was dissolved in a solution of 10% methanol and methylene chloride. Diazomethane was added and the solution was stirred for 1 hour at room temperature. The solution was extracted with 100% methylene chloride followed by methylene chloride +1% methanol. The desired product was combined from recrystallization (ethyl ether/hexane) and from the filtrate to yield 1.23g of 2-(3-chlorobenzoyl)-3-methoxycarbonylpyridine.

### EXAMPLE 14

### Preparation of 8-(3-chlorophenyl)pyrido[2,3.d]pyridazin-5-one

### 14. Formula I, Where X is nitrogen, Y is carbon, Z is oxygen, R¹ is hydrogen, R³ is chloro and R², R⁴, R⁵ and R⁶ are hydrogen.

A solution of 2-(3-chlorobenzoyl)-3-methoxycarbonylpyridine (0.55g, 2.0mmoles), hydrazine hydrate (0.15ml, 3.0mmoles) in ethanol (50ml) was stirred at 100°C for 18 hours under an inert atmosphere. The solution was cooled and solid in the solution was collected, washed with ethyl ether and dried to give 0.26g of 8-(3-chlorophenyl)pyrido[2,3-d]-pyridazin-5-one (50.5%), mp 268-269°C.

### EXAMPLE 15

### Preparation of 6-phenyl-8-(3-chlorophenyl)pyrido[2,3-d]pyridazin-5-one

### 15. Formula I, Where X is nitrogen, Y is carbon, Z is oxygen, R¹ is phenyl, R³ is chloro and R², R⁴, R⁵ and R⁶ are hydrogen.

A solution of 2-(3-chlorobenzoyl)-3-methoxycarbonylpyridine (0.30g, 1.09mmoles), phenylhydrazine (0.27ml, 2.72mmoles) in ethanol (18ml) was stirred at 100°C for 72 hours. The solution was cooled and the solvent was removed. The residue was triturated with methanol to give a white solid, which was collected and air dried, yielding 0.26g of 6-phenyl-8-(3-chlorophenyl)pyrido[2,3-d]pyridazin-5-one (71.5%), mp 144-145°C.

### EXAMPLE 16

### Preparation of 6-benzyl-8-(3-nitrophenyl)pyrido[2,3-d]pyridazin-5-one

### 16. Formula I, Where X is nitrogen, Y is carbon, Z is oxygen, R¹ is benzyl, R³ is chloro and R², R⁴, R⁵ and R⁶ are hydrogen.

A solution of 2-(3-chlorobenzoyl)-3-methoxycarbonylpyridine (0.30g, 1.09mmoles), benzylhydrazine hydrochloride (0.53ml, 2.72mmoles) in ethanol (18ml) was refluxed for 132 hours (5 days). The solution was cooled and the solvent was removed. The residue was dissolved in ethanol (minimal amount used) and stored in a freezer to induce crystallization. The solid material was collected from the solution, chromatographed, eluted with 100% methylene chloride, and crystallized from methanol to yield 0.21g of 6-benzyl-8-(3-nitrophenyl)pyrido[2,3-d]pyridazin-5-one (55.4%), mp 141°C.

### EXAMPLE 17

### Preparation of 6-ethyl-8-(3-chlorophenyl)pyrido[2,3-d]pyridazin-5-one

### 17. Formula I, Where X is nitrogen, Y is carbon, Z is oxygen, R¹ is ethyl, R³ is chloro and R², R⁴, R⁵ and R⁶ are hydrogen.

To a suspension of 8-(3-chlorophenyl)pyrido[2,3-d]pyridazin-5-one (0.32g, 1.24mmoles) in tetrahydrofuran (50ml) was added sodium hydride (0.06g, 1.4mmoles). The mixture was stirred for 1 hour at room temperature. Iodoethane (0.48g, 3.lmmoles) was added and the solution was refluxed for 18 hours under an inert atmosphere. The mixture was cooled and acidified by the addition of 50ml of 1N HCl. The desired compound was isolated by extraction with ethyl acetate (extracted until no product detected in aqueous layer), washed with sodium bicarbonate, brine and dried over magnesium sulfate. The solution was filtered and the solvent removed yielding 0.85g of the crude desired product. The product was purified by recrystallization from methanol to yield 0.24g of 6-ethyl-8-(3-chlorophenyl)pyrido[2,3-d]pyridazin-5-one (67.8%), mp. 117-118°C.

### EXAMPLE 18

### Preparation of 6-(2-propyl)-8-(3-chlorophenyl)pyrido[2,3-d]pyridazin-5-one

### 18. Formula I, Where X is nitrogen, Y is carbon, Z is oxygen, R¹ is 2-propyl, R³ is chloro and R², R⁴, R⁵ and R⁶ are hydrogen.

To a suspension of 8-(3-chlorophenyl)pyrido[2,3-d]pyridazin-5-one (0.33g, 1.28mmoles) in tetrahydrofuran (50ml) was added sodium hydride (0.07g, 1.53mmoles). The mixture was stirred at room temperature for 30 minutes under an inert atmosphere. 2-Iodopropane (0.64ml, 6.4mmoles) was added and the mixture refluxed for 48 hours. The mixture was cooled and acidified by the addition of 50ml of 1N HCl. The mixture was extracted with ethyl acetate (3x50ml) and dried over magnesium sulfate. The solution was filtered and the solvent removed to yield the crude product. The product was purified by crystallization (ethyl ether/ hexane) yielding 0.15g of 6-(2-propyl)-8-(3-chlorophenyl)pyrido[2,3-d]-pyridazin-5-one (39%), mp 80-81°C

### EXAMPLE 19

### Preparation of 6-(4-pyridylmethyl)-8-(3-chlorophenyl)pyrido[2,3-d]pyridazin-5-one

### 19. Formula I, Where X is nitrogen, Y is carbon, Z is oxygen, R¹ is 4-pyridylmethyl, R³ is chloro and R², R⁴, R⁵ and R⁶ are hydrogen.

To a suspension of 8-(3-chlorophenyl)pyrido[2,3-d]pyridazin-5-one(0.50g, 1.90mmoles) in dimethylformamide (50ml) was added potassium carbonate (3.22g, 23.3mmoles). The mixture was stirred at room temperature for 1 hour under an inert atmosphere.
4-Picolyl chloride (1.59g, 9.7mmoles) and potassium iodide (1.61g, 9.7mmoles) was added, the mixture was stirred at 80°C for 168 hours (7 days). The mixture was cooled, the insoluble material was filtered out and the solvent removed yielding 2.13g of the crude product. The product was purified by chromatography, eluted with 100% ethylacetate and crystallized from methanol yielding 0.091g of 6-(4-pyridylmethyl)-8-(3-chlorophenyl)pyrido[2,3-d]pyridazin-5-one (13.7%), mp 152°C.

### EXAMPLE 20

### Preparation of 2-(3-chlorobenzoyl)-3-carboxypyridine and 2-carboxy-3-(3-chlorobenzoyl)pyridine Using A Grignard Reagent

### 20. Formula 6, Where X is nitrogen, Y is carbon, R³ is chloro and R², R⁴, R⁵ and R⁶ are hydrogen.

A Grignard reagent was prepared by adding l-bromo-3-chlorobenzene (11.75ml, 100mmoles) to a suspension of magnesium (2.43g, 100mmoles) in tetrahydrofuran (100ml) and stirred at room temperature for 18 hours under an inert atmosphere. The Grignard reagent was added in a dropwise manner to a solution of 2,3-pyridyldicarboxylic anhydride (14.91ml, 100mmoles) in tetrahydrofuran (200ml) at a temperature of -78°C over a 2 hour period. After the addition the solution was stirred for an additional 45 minutes. 1N HCl (110ml) was added and the solution was stirred for 1 hour. The desired product was extracted with ethyl acetate, washed in brine and dried over Na₂SO₄ yielding 2-(3-chlorobenzoyl)-3-carboxypyridine and 2-carboxy-3-(3-chlorobenzoyl)pyridine.

2-(3-Chlorobenzoyl)-3-carboxypyridine (Formula 6, where X is nitrogen, Y is carbon, R³ is chloro and R², R⁴, R⁵ and R⁶ are hydrogen) can be converted to the corresponding compounds of Formulae II and I by following the procedures of Examples 13-19, respectively.

### EXAMPLE 21

### Preparation of 2-methoxycarbonyl-3-(3-chlorobenzoyl)pyridine

### 21. Formula II, Where X is carbon and Y is nitrogen, R³ is chloro, and R², R⁴, R⁵ and R⁶ are hydrogen.

Following the procedures of Example 13 and substituting 2-(3-chlorobenzoyl)-3-carboxypyridine with 2-carboxy-3-(3-chlorobenzoyl)-pyridine there is obtained 2-methoxycarbonyl-3-(3-chlorobenzoyl)-pyridine.

### EXAMPLE 22

### Preparation of 5-(3-chlorophenyl)pyrido[2,3-d]pyridazin-8-one

### 22. Formula I, Where X is carbon, Y is nitrogen, Z is oxygen, R³ is chloro and R¹, R², R⁴, R⁵ and R⁶ are hydrogen.

Following the procedures of Example 14 and substituting 2-(3-chlorobenzoyl)-3-methoxycarbonylpyridine with 2-methoxycarbonyl-3-(3-chlorobenzoyl)pyridine there is obtained 5-(3-chlorophenyl)pyrido-[2,3-d]pyridazin-8-one.

### EXAMPLE 23

### Preparation of 5-(3-chlorophenyl)-7-phenylpyrido[2,3-d]pyridazin-8-one

### 23. Formula I, Where X is carbon, Y is nitrogen, Z is oxygen, R¹ is phenyl, R³ is chloro and R², R⁴, R⁵ and R⁶ are hydrogen.

Following the procedures of Example 15 and substituting 2-(3-chlorobenzoyl)-3-methoxycarbonylpyridine with 2-methoxycarbonyl-3-(3-chlorobenzoyl)pyridine there is obtained 5-(3-chlorophenyl)-7-phenylpyrido[2,3-d]pyridazin-8-one.

### EXAMPLE 24

### Preparation of 5-(3-chlorophenyl)-7-benzylpyrido[2,3-d]pyridazin-8-one

### 24. Formula I, Where X is carbon, Y is nitrogen, Z is oxygen, R¹ is benzyl, R³ is chloro and R², R⁴, R⁵ and R⁶ are hydrogen.

Following the procedures of Example 16 and substituting 2-(3-chlorobenzoyl)-3-methoxycarbonylpyridine with 2-methoxy carbonyl-3-(3-chlorobenzoyl)pyridine there is obtained 5-(3-chlorophenyl)-7-benzylpyrido[2,3-d]pyridazin-8-one.

### EXAMPLE 25

### Preparation of 5-(3-chlorophenyl)-7-ethylpyrido[2,3-d]pyridazin-8-one

### 25. Formula I, Where X is carbon, Y is nitrogen, Z is oxygen, R¹ is ethyl, R³ is chloro and R², R⁴, R⁵ and R⁶ are hydrogen.

Following the procedures of Example 17 and substituting 8-(3-chlorophenyl)pyrido[2,3-d]pyridazin-5-one with 5-(3-chlorophenyl)-pyrido[2,3-d]pyridazin-8-one there is obtained 5-(3-chlorophenyl)-7-ethylpyrido[2,3-d]pyridazin-8-one.

### EXAMPLE 26

### Preparation of 5-(3-chlorophenyl)-7-(2-propyl)pyrido[2,3-d]pyridazin-8-one

### 26. Formula I, Where X is carbon, Y is nitrogen, Z is oxygen, R¹ is 2-propyl, R³ is chloro and R², R⁴, R⁵ and R⁶ are hydrogen.

Following the procedures of Example 18 and substituting 8-(3-chlorophenyl)pyrido[2,3-d]pyridazin-5-one with 5-(3-chlorophenyl)pyrido[2,3-d]pyridazin-8-one there is obtained 5-(3-chlorophenyl)-7-(2-propyl)pyrido[2,3-d]pyridazin-8-one.

### EXAMPLE 27

### Preparation of 5-(3-chlorophenyl)-7-(4-pyridylmethyl)pyrido[2,3-d]pyridazin-8-one

### 27. Formula I, Where X is carbon, Y is nitrogen, Z is oxygen, R¹ is 4-pyridylmethyl, R³ is chloro and R², R⁴, R⁵ and R⁶ are hydrogen.

Following the procedures of Example 19 and substituting 8-(3-chlorophenyl)pyrido[2,3-d]pyridazin-5-one with 5-(3-chlorophenyl)-pyrido[2,3-d]pyridazin-8-one there is obtained 5-(3-chloro phenyl)-7-(4-pyridomethyl)pyrido[2,3-d]pyridazin-8-one.

### EXAMPLE 28

### Preparation of 2-Methoxycarbonyl-3-benzoylpyridine

### 28. Formula II, Where X is carbon, Y is nitrogen and R², R³, R⁴, R⁵ and R⁶ are hydrogen.

A solution of 2,3-pyridyldicarboxylic anhydride (10 g, 67.1 mmoles) in dry benzene (80 ml, 888 mmoles) was stirred at 50-55°C while aluminum chloride (AlCl₃, 20 g, 148 mmoles) was gradually added. The mixture was refluxed for 18 hours. The reaction mixture was cooled and poured into 250 ml of 5% aqueous HCl and extracted with ethyl acetate. The ethyl acetate was removed and the residue was dried under vacuum. The residue was dissolved in 500 ml methanol and 4 ml concentrated H₂SO₄ (67.1 mmoles) was added. The solution was refluxed at 80°C for 24 hours. The mixture was cooled, neutralized with saturated sodium bicarbonate solution and extracted with ethyl acetate. The ethyl acetate extract was washed with saturated brine and dried over sodium sulfate. The solution was filtered and the ethyl acetate removed under vacuum to yield 2-methoxycarbonyl-3-benzoylpyridine (7.92 g, 32.8 mmoles, 49% crude yield). The product was purified by column chromatography eluting with 5% ethyl acetate in methylene chloride to give after crystallization from ether the purified product (5.61 g, 23 mmoles, 35% yield), mp 85-86°C.

### EXAMPLE 29

### Preparation of 2-(3-nitrobenzoyl)-3-methoxycarbonylpyridine

### 29. Formula II, Where X is nitrogen, Y is carbon, R³ is nitro, and R², R⁴, R⁵ and R⁶ are hydrogen.

2-Benzoyl-3-methoxycarbonylpyridine (3.86 g, 16.0 mmoles) was dissolved in H₂SO₄ (10 ml) to which a mixture of HNO₃ (0.88 ml) and H₂SO₄ (2.0 ml) was added dropwise. The reaction mixture was stirred for 30 minutes and poured onto ice. The solution was stirred for 60 minutes, basified to pH 7.8 with saturated sodium bicarbonate and extracted with ethyl acetate. The organic ethyl acetate solution was washed with brine and dried over magnesium sulfate. The solution was filtered and the solvent removed yielding 4.49 g of the crude product, 2-(3-nitrobenzoyl)-3-methoxycarbonylpyridine.

### EXAMPLE 30

### Preparation of 5-(3-nitrophenyl)-pyrido[2,3-d]pyridazin-8-one

### 30. Formula I, Where X is carbon, Y is nitrogen, Z is oxygen, R³ is nitro and R¹, R², R⁴, R⁵ and R⁶ are hydrogen.

Following the procedures of Example 5 and substituting 2-(3-nitrobenzoyl)-3-methoxycarbonylpyridine with 2-methoxycarbonyl-3-(3-nitrobenzoyl)pyridine, as prepared according to the procedure of Example 29B, there is obtained 5-(3-nitrophenyl)pyrido[2,3-d]pyridazin-8-one.

### EXAMPLE 31

### Preparation of 5-(3-nitrophenyl)-7-phenylpyrido[2,3-d]pyridazin-8-one

### 31. Formula I, Where X is carbon, Y is nitrogen, Z is oxygen, R¹ is phenyl, R³ is nitro and R², R⁴, R⁵ and R⁶ are hydrogen.

Following the procedures of Example 6 and substituting 2-(3-nitrobenzoyl)-3-methoxycarbonylpyridine with 2-methoxycarbonyl-3-(3-nitrobenzoyl)pyridine there is obtained 5-(3-nitrophenyl)-7-phenylpyrido[2,3-d]pyridazin-8-one.

### EXAMPLE 32

### Preparation of 5-(3-nitrophenyl)-7-benzylpyrido[2,3-d]pyridazin-8-one

### 32. Formula I, Where X is carbon, Y is nitrogen, Z is oxygen, R¹ is benzyl, R³ is nitro and R², R⁴, R⁵ and R⁶ are hydrogen.

Following the procedures of Example 7 and substituting 2-(3-nitrobenzoyl)-3-methoxycarbonylpyridine with 2-methoxycarbonyl-3-(3-nitrobenzoyl)pyridine there is obtained 5-(3-nitrophenyl)-7-benzylpyrido[2,3-d]pyridazin-8-one.

### EXAMPLE 33

### Preparation of 5-(3-nitrophenyl)-7-ethylpyrido[2,3-d]pyridazin-8-one

### 33. Formula I, Where X is carbon, Y is nitrogen, Z is oxygen, R¹ is ethyl, R³ is nitro and R², R⁴, R⁵ and R⁶ are hydrogen.

Following the procedures of Example 8 and substituting 8-(3-nitrophenyl)pyrido[2,3-d]pyridazin-5-one with 5-(3-nitrophenyl)pyrido[2,3-d]pyridazin-8-one there is obtained 5-(3-nitrophenyl)-7-ethylpyrido[2,3-d]pyridazin-8-one.

### EXAMPLE 34

### Preparation of 5-(3-nitrophenyl)-7-(4-pyridylmethyl)pyrido[2,3-d]pyridazin-8-one

### 34. Formula I, Where X is carbon, Y is nitrogen, Z is oxygen, R¹ is 4-pyridylmethyl, R³ is nitro and R², R⁴, R⁵ and R⁶ are hydrogen.

Following the procedures of Example 9 and substituting 8-(3-nitrophenyl)pyrido[2,3-d]pyridazin-5-one with 5-(3-nitrophenyl)pyrido[2,3-d]pyridazin-8-one there is obtained 5-(3-nitrophenyl)-7-(4-pyridomethyl)pyrido[2,3-d]pyridazin-8-one.

### EXAMPLE 35

### Preparation of 6-(3-thienylmethyl)-8-(3-chlorophenyl)pyrido[2,3-d]pyridazin-5-one

### 35A. Formula I, Where X is nitrogen, Y is carbon, Z is oxygen, R¹ is 3-thiophenemethyl, R³ is chloro and R², R⁴, R⁵ and R⁶ are hydrogen.

To a suspension of 8-(3-chlorophenyl)pyrido[2,3-d]pyridazin-5-one (0.21g, 0.82mmoles) in tetrahydrofuran (25ml) was added 3-thiophenemethanol (0.085ml, 0.9 mmoles) and triphenyl phosphine (0.32g, 1.22 mmoles). To this mixture was added a solution of diisopropyl azodicarboxylate (0.24ml, 1.22 mmoles) in tetrahydrofuran (5ml) in a dropwise manner. The solution was stirred for 3 hours at room temperature. The solvent was removed and the residue chromatographed from hot methanol, yielding 0.15 g of 6-(3-thienylmethyl)-8-(3-chlorophenyl)pyrido[2,3-d]pyridazin-5-one, mp 165-166°C.

### 35B. Preparation of Other Compounds of Formula I Where X is nitrogen, Y is carbon, and Z is oxygen.

Following the procedures of Example 35A and substituting 3-thiophenemethanol with the following:
4-(2-hydroxyethyl)morpholine;
3-pyridinepropanol;
2-(hydroxyethyl)pyridine;
1-(2-hydroxyethyl)pyrrolidine; and
1-(2-hydroxyethyl)-2-pyrrolidinone,
their were obtained the following compounds:
6-[4-(2-hydroxyethyl)morpholinyl]-8-(3-chlorophenyl)pyrido[2,3-d]pyridazin-5-one, mp 135°C;
**6-(3-pyridopropyl)-8-(3-chlorophenyl)pyrido[2,3-d]**-pyridazin-5-one, mp 91°C;
6-[2-(hydroxyethyl)pyridyl]-8-(3-chlorophenyl)pyrido[2,3-d]pyridazin-5-one, mp 98°C;
6-[1-(2-hydroxyethyl)pyrrolidyl]-8-(3-chlorophenyl)pyrido[2,3-d]pyridazin-5-one, mp 149°C; and
6-[1-(2-hydroxyethyl)-2-pyrrolidinonyl]-8-(3-chlorophenyl)pyrido[2,3-d]pyridazin-5-one, mp 78°C.

### EXAMPLE 36

### Preparation of 6-(4-pyridylmethyl)-8-(3-nitrophenyl)pyrido[2,3-d]pyridazin-5-one hydrochloride

To a suspension of 6-(4-pyridylmethyl)-8-(3-nitrophenyl)pyrido[2,3-d]pyridazin-5-one in methanol is added 5% HCl/MeOH in a dropwise manner with stirring until a clear solution is obtained. The solvent is stripped off. The resulting solid is triturated with ethyl ether. The product is filtered off and dried under vacuum yielding 6-(4-pyridylmethyl)-8-(3-nitrophenyl)pyrido[2,3-d]pyridazin-5-one hydrochloride.

In a similar manner, all compounds of Formula I may be converted to the acid addition salts by treatment with the appropriate acid, for example, hydrobromic acid, sulfuric acid (giving the sulfate and bisulfate salts), nitric acid, phosphoric acid and the like.

### EXAMPLE 37

By following the procedures in Examples 1-36 the following compounds were made:
**Formula I, where X is nitrogen, Y is carbon, and Z is oxygen.**
R³ is chloro, R¹ is 2-propyl, R², R⁴, R⁵ and R⁶ are hydrogen, melting point 80°C;
R³ is chloro, R¹ is 4-pyridylmethyl, R², R⁴, R⁵ and R⁶ are hydrogen, melting point 152°C;
R³ is chloro, R¹ is N-ethylmorpholinyl, R², R⁴, R⁵ and R⁶ are hydrogen, melting point 135 °C;
R³ is chloro, R¹ is N-ethylpyrrolidonyl, R², R⁴, R⁵ and R⁶ are hydrogen, melting point 78 °C;
R³ is chloro, R¹ is 3-thienylmethyl, R², R⁴, R⁵ and R⁶ are hydrogen, melting point 165°C;
R³ is chloro, R¹ is (4-methoxy-3-pyridyl)methyl, R², R⁴, R⁵ and R⁶ are hydrogen, melting point 145°C;
R³ is chloro, R¹ is 3-pyridylmethyl, R², R⁴, R⁵ and R⁶ are hydrogen, melting point 165°C;
R³ is chloro, R¹ is n-butyl, R², R⁴, R⁵ and R⁶ are hydrogen, melting point 119°C;
R³ is chloro, R¹ is methyl, R², R⁴, R⁵ and R⁶ are hydrogen, melting point 180°C;
R³ is chloro, R¹ is benzyl, R², R⁴, R⁵ and R⁶ are hydrogen, melting point 111-112°C;
R³ is chloro, R¹ is ethyl, R², R⁴, R⁵ and R⁶ are hydrogen, melting point 117°C;
R³ is chloro, R¹, R², R⁴, R⁵ and R⁶ are hydrogen, melting point 268-269°C;
R³ is chloro, R¹ is phenyl, R², R⁴, R⁵ and R⁶ are hydrogen, melting point 144-145°C;
R³ is chloro, R¹ is N-methylpiperidinyl, R², R⁴, R⁵ and R⁶ are hydrogen, melting point 142°C;
R³ is chloro, R¹ is 2-pyridylethyl, R², R⁴, R⁵ and R⁶ are hydrogen, melting point 98°C;
R³ is chloro, R¹ is 3-pyridylpropyl, R², R⁴, R⁵ and R⁶ are hydrogen, melting point 91°C;
R³ is chloro, R¹ is phenylethyl, R², R⁴, R⁵ and R⁶ are hydrogen, melting point 108°C;
R³ is chloro, R¹ is cyclopentyl, R², R⁴, R⁵ and R⁶ are hydrogen, melting point 93°C;
R³ is chloro, R¹ is cyclohexyl, R², R⁴, R⁵ and R⁶ are hydrogen, melting point 98°C;
R³ is chloro, R¹ is N-ethylpyrrolidinyl, R², R⁴, R⁵ and R⁶ are hydrogen, melting point 149°C;
R³ is chloro, R¹ is n-propyl, R², R⁴, R⁵ and R⁶ are hydrogen, melting point 93°C;
R³ is chloro, R¹ is t-butyl, R², R⁴, R⁵ and R⁶ are hydrogen, melting point 105°C;
R³ is chloro, R¹ is N-methylmorpholinyl, R², R⁴, R⁵ and R⁶ are hydrogen, melting point 161°C;
R³ is chloro, R¹ is 2-methylaminoethyl and R², R⁴, R⁵ and R⁶ are hydrogen, melting point 90°C.
R³ is chloro, R¹ is cyclopentylmethyl and R², R⁴, R⁵ and R⁶ are hydrogen, melting point 125°C.
R³ is chloro, R¹ is α-methyl-cyclopropylmethyl and R², R⁴, R⁵ and R⁶ are hydrogen, melting point 76°C.
R³ is chloro, R¹ is 2-thienyl and R², R⁴, R⁵ and R⁶ are hydrogen, melting point 175°C.
R³ is chloro, R¹ is furfuryl and R², R⁴, R⁵ and R⁶ are hydrogen, melting point 164°C.
R³ is chloro, R¹ is 4-pyridyl-N-oxide-methyl and R², R⁴, R⁵ and R⁶ are hydrogen, melting point 175-176°C (recryst. from MeOH).
R³ is chloro, R¹ is dimethylaminoethyl and R², R⁴, R⁵ and R⁶ are hydrogen, melting point 90°C.
R³ and R⁴ are chloro, R¹ is hydrogen and R², R⁵ and R⁶ are hydrogen, melting point 270-272°C.
R³ and R⁴ are chloro, R¹ is ethyl and R², R⁵ and R⁶ are hydrogen, melting point 114°C.
R³ and R⁴ are chloro, R¹ is isopropyl and R², R⁵ and R⁶ are hydrogen, melting point 133°C.
R³ and R⁴ are chloro, R¹ is benzyl and R², R⁵ and R⁶ are hydrogen, melting point 145°C.
R³ and R⁴ are chloro, R¹ is 4-pyridylmethyl and R², R⁵ and R⁶ are hydrogen, melting point 162°C.
R³ is fluoro, R¹ is 4-pyridylmethyl and R², R⁴, R⁵ and R⁶ are hydrogen, melting point 158°C.
R³ is fluoro, R¹ is 3-pyridylmethyl and R², R⁴, R⁵ and R⁶ are hydrogen, melting point 151°C.
R³ is fluoro, R¹ is ethyl and R², R⁴, R⁵ and R⁶ are hydrogen, melting point 117-118°C.
R³ is fluoro, R¹ is benzyl and R², R⁴, R⁵ and R⁶ are hydrogen, melting point 130-131°C.
R³ is nitro, R¹ is 4-pyridylmethyl, R², R⁴, R⁵ and R⁶ are hydrogen, melting point 172°C;
R³ is nitro, R¹ is 4-pyridylmethyl, R², R⁴, R⁵ and R⁶ are hydrogen, as hydrochloride; melting point 195.9-203.9°C;
R³ is nitro, R¹ is 3-pyridylmethyl, R², R⁴, R⁵ and R⁶ are hydrogen, melting point 190°C;
R³ is nitro, R¹ is 3-thienylmethyl, R², R⁴, R⁵ and R⁶ are hydrogen, melting point 175°C;
R³ is nitro, R¹ is phenyl, R², R⁴, R⁵ and R⁶ are hydrogen, melting point 167-168°C;
R³ is nitro, R¹ is ethyl, R², R⁴, R⁵ and R⁶ are hydrogen, melting point 138-139°C;
R³ is nitro, R¹ is allyl, R², R⁴, R⁵ and R⁶ are hydrogen, melting point 99°C;
R³ is nitro, R¹, R², R⁴, R⁵ and R⁶ are hydrogen, melting point 302-304°C;
R³ is nitro, R¹ is benzyl, R², R⁴, R⁵ and R⁶ are hydrogen, melting point 141°C;
R³ is nitro, R¹ is 2-methoxybenzyl, R², R⁴, R⁵ and R⁶ are hydrogen, melting point 189°C;
R³ is nitro, R¹ is 3-fluorobenzyl, R², R⁴, R⁵ and R⁶ are hydrogen, melting point 139°C;
R³ is nitro, R¹ is 3-methoxybenzyl, R², R⁴, R⁵ and R⁶ are hydrogen, melting point 162°C;
R³ is nitro, R¹ is 3,5-dichlorobenzyl, R², R⁴, R⁵ and R⁶ are hydrogen, melting point 189°C;
R³ is nitro, R¹ is 4-nitrobenzyl, R², R⁴, R⁵ and R⁶ are hydrogen, melting point 205°C;
R³ is nitro, R¹ is 4-fluorobenzyl, R², R⁴, R⁵ and R⁶ are hydrogen, melting point 155°C;
R³ is nitro, R¹ is 4-chlorobenzyl, R², R⁴, R⁵ and R⁶ are hydrogen, melting point 177°C;
R³ is nitro, R¹ is 4-methoxybenzyl, R², R⁴, R⁵ and R⁶ are hydrogen, melting point 155°C;
R³ is nitro, R¹ is cyclopentylmethyl, R², R⁴, R⁵ and R⁶ are hydrogen, melting point 88°C;
R³ is nitro, R¹ is dimethylaminoethyl, R², R⁴, R⁵ and R⁶ are hydrogen, melting point 108°C;
R³ is nitro, R¹ is 2-tetrahydrofuranylmethyl, R², R⁴, R⁵ and R⁶ are hydrogen, melting point 109°C;
R³ is nitro, R¹ is 3-tetrahydrofuranylmethyl, R², R⁴, R⁵ and R⁶ are hydrogen, melting point 76°C;
R³ is nitro, R¹ is cyclopropylmethyl, R², R⁴, R⁵ and R⁶ are hydrogen, melting point 129-130°C;
R³ is nitro, R¹ is α-methyl-cyclopropylmethyl, R², R⁴, R⁵ and R⁶ are hydrogen, melting point 99°C;
R³ is nitro, R¹ is 4-pyridyl-N-oxide-methyl, R², R⁴, R⁵ and R⁶ are hydrogen, melting point 208°C;
R³ is nitro, R¹ is 2-propyl, R², R⁴, R⁵ and R⁶ are hydrogen, melting point 126°C;
R³ is nitro, R¹ is 1,2-(methylenedioxy)phenyl-5-methyl, R², R⁴, R⁵ and R⁶ are hydrogen, melting point 199°C;
R³ is nitro, R¹ is 2-bromoethyl, R², R⁴, R⁵ and R⁶ are hydrogen, melting point 163°C;
R³ is nitro, R¹ is 2-fluoroethyl, R², R⁴, R⁵ and R⁶ are hydrogen, melting point 167°C;
R³ is nitro, R¹ is 2-chloroethyl, R², R⁴, R⁵ and R⁶ are hydrogen, melting point 166°C;
R³ is nitro, R¹ is (3-methyl-3-oxetanyl)methyl, R², R⁴, R⁵ and R⁶ are hydrogen, melting point 159°C;
R³ is nitro, R¹ is 2-cyanoethyl, R², R⁴, R⁵ and R⁶ are hydrogen, melting point 145°C;
R³ is nitro, R¹ is 3-methyl-3-butenyl, R², R⁴, R⁵ and R⁶ are hydrogen, melting point 97°C;
R³ is nitro, R¹ is methylthioethyl, R², R⁴, R⁵ and R⁶ are hydrogen, melting point 96°C;
R³ is nitro, R¹ is 3-methyl-2-butenyl, R², R⁴, R⁵ and R⁶ are hydrogen, melting point 119°C;
R³ is nitro, R¹ is methoxyethyl, R², R⁴, R⁵ and R⁶ are hydrogen, melting point 135°C;
R³ is nitro, R¹ is methylthiopropyl, R², R⁴, R⁵ and R⁶ are hydrogen, melting point 71°C;
R³ is nitro, R¹ is 3,4-dichlorophenyl, R², R⁴, R⁵ and R⁶ are hydrogen, melting point 168°C;
R³ is nitro, R¹ is 2-hydroxyethyl, R², R⁴, R⁵ and R⁶ are hydrogen, melting point 155°C;
R³ is nitro, R¹ is 2-thienyl, R², R⁴, R⁵ and R⁶ are hydrogen, melting point 173°C;
R³ is nitro, R¹ is benzyl, R², R⁴, R⁵ and R⁶ are hydrogen, melting point 141°C;
R³ is nitro, R¹ is 1-pyrrolidinylethyl, R², R⁴, R⁵ and R⁶ are hydrogen, melting point 149°C;
R³ is nitro, R¹ is furfuryl, R², R⁴, R⁵ and R⁶ are hydrogen, melting point 159°C;
R³ is nitro, R¹ is 2-acetoxyethyl, R², R⁴, R⁵ and R⁶ are hydrogen, melting point 116°C;
R³ is nitro, R¹ is 2-(4-chlorophenyl)-4-thiazolylmethyl, R², R⁴, R⁵ and R⁶ are hydrogen, melting point 206°C;
R³ is nitro, R¹ is 2-phenyl-4-oxazolylmethyl, R², R⁴, R⁵ and R⁶ are hydrogen, melting point 170°C;
R³ is nitro, R¹ is 5-norbornen-2-yl-methyl, R², R⁴, R⁵ and R⁶ are hydrogen, melting point 152°C;
R³ is nitro, R¹ is norbornylmethyl, R², R⁴, R⁵ and R⁶ are hydrogen, melting point 153°C;
R³ is nitro, R¹ is 3-[5-(3,5-dimethylisoxazol-4-yl)-1,2,4-oxadiazolyl)]methyl, R², R⁴, R⁵ and R⁶ are hydrogen, melting point 190°C;
R³ is trifluoromethyl, R¹ is cyanoethyl, R², R⁴, R⁵ and R⁶ are hydrogen, melting point 133°C;
R³ is trifluoromethyl, R¹ is ethyl, R², R⁴, R⁵ and R⁶ are hydrogen, melting point 116°C;
R³ is trifluoromethyl, R¹ is 3-pyridylmethyl, R², R⁴, R⁵ and R⁶ are hydrogen, melting point 142°C;
R³ is trifluoromethyl, R¹ is benzyl, R², R⁴, R⁵ and R⁶ are hydrogen, melting point 137°C;
R³ is trifluoromethyl, R¹ is n-butyl, R², R⁴, R⁵ and R⁶ are hydrogen, melting point 103°C;
R³ is cyano, R¹ is benzyl, R², R⁴, R⁵ and R⁶ are hydrogen, melting point 158°C;
R³ is methyl, R¹ is benzyl, R², R⁴, R⁵ and R⁶ are hydrogen, melting point 88°C;
R³ is methoxycarbonyl, R¹ is benzyl, R², R⁴, R⁵ and R⁶ are hydrogen, melting point 128-130°C;
R¹ is benzyl, R², R³, R⁴, R⁵ and R⁶ are hydrogen, melting point 169-170°C;
R¹ is 4-pyridylmethyl, R², R³, R⁴, R⁵ and R⁶ are hydrogen, melting point 175-176°C;
R¹, R², R³, R⁴, R⁵ and R⁶ are hydrogen, melting point 230-231°C;
R¹ is ethyl, R³, R², R⁴, R⁵ and R⁶ are hydrogen, melting point 114-115°C;
**Formula I, Where X is carbon, Y is nitrogen, and Z is oxygen.**
R¹ is phenyl, R², R³, R⁴, R⁵ and R⁶ are hydrogen, having a melting point of 221-222°C;
R¹, R², R³, R⁴, R⁵ and R⁶ are hydrogen, having a melting point of 232-233°C;
R¹ is ethyl, R², R³, R⁴, R⁵ and R⁶ are hydrogen, having a melting point of 164-165°C;
R¹ is benzyl, R², R³, R⁴, R⁵ and R⁶ are hydrogen, having a melting point of 210-211°C; and
R¹ is 4-pyridylmethyl, R², R³, R⁴, R⁵ and R⁶ are hydrogen, having a melting point of 230-231°C.
**Formula I, where X is nitrogen, Y is carbon, and Z is sulfur.**
R³ is chloro, R¹ is benzyl, R², R⁴, R⁵ and R⁶ are hydrogen, melting point 132°C;

### EXAMPLE 38

### Preparation of 6-(4-pyridyl-N-oxide-methyl)-8-(3-chlorophenyl)pyrido[2,3-d]pyridazin-5-one

### 38. Formula I, where X is nitrogen, Y is carbon, Z is oxygen, R¹ is 4-pyridyl-N-oxide-methyl, R³ is chloro and R², R⁴, R⁵ and R⁶ are hydrogen.

To a suspension of 8-(3-chlorophenyl)pyrido[2,3-d]pyridazin-5-one (0.50 g, 1.94 mmoles), 4-pyridylcarbinol N-oxide (0.27 g, 2.13 mmoles), triphenylphosphine (0.76 g, 2.9 mmoles) in tetrahydrofuran (50 ml) was added diisopropylazodicarboxylate (0.57 ml, 2.9 mmoles) in a dropwise fashion. The suspension was stirred under nitrogen for 18 hours at room temperature. The solvent was removed and the product purified by chromatography, eluted with 100% ethylacetate and 10% methanol/ ethylacetate to yield 0.382 g of 6-(4-pyridyl-N-oxide-methyl)-8-(3-chlorophenyl)pyrido[2,3-d]pyridazin-5-one (54%), mp 99°C.

### EXAMPLE 39

### Preparation of 6-(4-pyridyl-N-oxide-methyl)-8-(3-nitrophenyl)pyrido[2,3-d]pyridazin-5-one

### 39. Formula I, where X is nitrogen, Y is carbon, Z is oxygen, R¹ is 4-pyridyl-N-oxide-methyl, R³ is nitro and R², R⁴, R⁵ and R⁶ are hydrogen.

To a solution of 8-(3-nitrophenyl)pyrido[2,3-d]pyridazin-5-one (0.40 g, 1.5 mmoles), 4-pyridylcarbinol N-oxide (0.205 g, 1.64 mmoles), triphenylphosphine (0.587 g, 2.24 mmoles) in tetrahydrofuran (50 ml) was added diisopropylazodicarboxylate (0.44 ml, 2.24 mmoles) in a dropwise fashion. The suspension was stirred under nitrogen for 18 hours at room temperature. The solvent was removed and the product purified by chromatography, i.e., eluted with 100% ethylacetate and 10% methanol/ ethylacetate. The fractions were combined, the solvent removed yielding 0.390 g of product which was recrystallized from methanol to give 0.237 g of 6-(4-pyridyl-N-oxide-methyl)-8-(3-nitrophenyl)pyrido-[2,3-d]pyridazin-5-one (42.1%),

### EXAMPLE 40

### (Reaction Scheme F)

### Alternate Preparation of Formula 11, where X is nitrogen, Y is carbon and R⁷ is n-butyl.

A suspension of 2,3-pyridinyldicarboxylic anhydride (Formula 1) (90g, 603mmoles) was prepared with 400mL of ethyl acetate. The mixture was cooled to 3°C and n-butylamine (61mL, 610mmoles) was added at such a rate that the temperature remained below 25°C. Following the addition, the cooling bath was removed and thionyl chloride (51mL; 700mmoles) was added at such a rate that the temperature remained below 50°C. The mixture was stirred for 1 hour. The resulting suspension was then cooled to 5°C and dilute sodium hydroxide (240mL of a 6M solution, 1.4moles) was added at such a rate that the temperature remained below 35°C. The resulting layers were separated. The bulk of the ethyl acetate was removed by atmospheric distillation. Methanol (500mL) was then added and the distillation continued until 250mL of solution remained. The solution was allowed to cool during which time crystallization occurred. Water (250mL) was then added and the mixture aged for 18 hours. The product was isolated by filtration and washed twice with 200mL of a 1:1 (v:v) mixture of water and methanol. After drying 115g of product (Formula 11) was obtained.

### Preparation of Formula 13, where X is nitrogen, Y is carbon, R⁷ is n-butyl, and R², R³, R⁴, R⁵, R⁶ are hydrogen.

The imide (formula 11) (40.4g, 198mmoles) was dissolved in toluene (1L) and cooled to -65°C. Phenylmagnesium chloride (100mL, 2M in THF, 200mmoles) was added over 15 minutes. The cooling bath was then removed and the mixture allowed to warm to -10°C. The reaction was quenched by addition of 25mL of saturated ammonium chloride solution. The room temperature solution was diluted with 500mL of ethyl acetate and 500mL of water. The layers were separated. Removal of most of the organic solvent was accomplished by atmospheric distillation. When the remaining volume reached 150mL the mixture was cooled to below 80°C and 1L of hexanes was added. The resulting crystalline mass was filtered and dried to give 50.6g of product (Formula 13).

### Preparation of Formula 14, where X is nitrogen, Y is carbon, R⁷ is n-butyl, R³ is nitro, and R², R³, R⁴, R⁵, R⁶ are hydrogen.

The product from the Grignard addition (47.15g, 167mmoles) was mixed with 250mL of concentrated sulfuric acid (96%). The resulting mixture was placed in a cooling bath. Nitric acid (70%, 12mL, 189mmoles) was added at such a rate that the temperature remains below 45°C. The mixture was stirred for 30 minutes poured onto ice (500g) and diluted with 1L of water. The mixture was extracted twice with 700mL of ethyl acetate. The organic solvent was removed and the reaction product used directly in the next reaction.

### Preparation of Formula 15, where X is nitrogen, Y is carbon, R³ is nitro, and R², R⁴, R⁵, R⁶ are hydrogen.

The crude material from the preceding reaction was mixed with 500mL of water and 250mL of concentrated hydrochloric acid was added. The mixture was heated under reflux for 20 hours. After cooling the mixture was diluted with 500mL of water and the solid was removed by filtration. After washing the solid with water (500mL) and methanol (200mL) the material was dried to give 39.85g of product.

### Preparation of Formula I, where X is nitrogen, Y is carbon, R³ is nitro, and R¹, R², R⁴, R⁵, R⁶ are hydrogen.

The material from the preceding reaction (38.65g, 142mmoles) was mixed with 800mL of methanol. To this heterogenous mixture was added 35mL of anhydrous hydrazine. The resulting solution was then heated under reflux for 5 hours. After a 17 hour aging at room temperature the product was isolated by filtration. Washing the crystalline mass twice with methanol (250mL) and drying yielded 25.6g of product.

### Preparation of Formula I, where X is nitrogen, Y is carbon, R¹ is 4-pyridylmethyl, R³ is nitro, and R², R⁴, R⁵, R⁶ are hydrogen.

The material from the preceding reaction (5.12g, 19.lmmoles), sodium hydride (50% in oil, lg, 20.1mmoles) were mixed in 100mL of 1-methyl-2-pryrrolidone and the mixture heated to 65°C. In a separatory funnel, 4-picolyl chloride hydrochloride [(4-chloromethyl)pyridine hydrochloride] (4.5g, 27.4mmoles), 20mL of saturated potassium carbonate and 75mL of toluene are mixed. After separation, the toluene solution was added to the reaction mixture dropwise over the period of 1 hour. The reaction mixture was diluted with water (250mL) and extracted twice with ethyl acetate (200mL). After concentrating the organic fractions, methanol and hexanes (75mL each) were added and the mixture allowed to stir at room temperature for 17 hours. The material was filtered and dried to yield 5.2g of product.

### Preparation of Formula I, where X is nitrogen, Y is carbon, R¹ is 4-pyridyl-N-oxide-methyl, R³ is nitro, and R², R⁴, R⁵, R⁶ are hydrogen,

The material from the preceding reaction (4.2g, 11.5mmoles) was dissolved in 100mL of methylene chloride. To this solution was added m-chloroperoxybenzoic acid (85%, 4.2g, 20.7mmoles). The solution was stirred for 17 hours. After partitioning between methylene chloride and potassium carbonate the organic layer was directly chromatographed using silica gel. The product was eluted with 20% methanol in ethyl acetate. Recrystallization of the solid material obtained after solvent removal from methanol (200mL) gave after filtering 3.2g of product.

### EXAMPLE 41

### (Reaction Scheme G)

### Preparation of Formula 18, where X is nitrogen, Y is carbon, R¹ is n-butyl, R³ is chlorine and R², R⁴, R⁵, R⁶ are hydrogen.

The imide (Formula 11) (20g, 98mmoles) was dissolved in toluene(500mL) and cooled to -65°C. To this mixture was added 3-chlorophenylmagnesium bromide (prepared by heating under reflux 2.5g magnesium metal, 20g of 3-chlorobromobenzene and 100mL of tetrahydrofuran for 1 hour) at such a rate that the temperature of the reaction remained below -40°C. After the addition was complete, the cooling bath was removed and the mixture allowed to warm to 0°C. The reaction mixture was quenched with aqueous ammonium chloride (25mL) diluted with ethyl acetate (250mL) and water (250mL). After separating the resulting 2 layers, the organic fraction was dried with sodium sulfate (50g). Filtration, followed by solvent removal gave an oil which was used directly in the next reaction.

### Preparation of Formula 19, where X is nitrogen, Y is carbon, R³ is chlorine, and R², R⁴, R⁵, R⁶ are hydrogen.

To the crude material from the preceding reaction was added 500mL of water, 250mL of concentrated hydrochloric acid and the mixture was heated under reflux for 24 hours. The solution was then filtered, cooled and made basic with sodium hydroxide. An ethyl acetate extraction removed unhydrolyzed starting material and the pH of the resulting aqueous fraction was adjusted to pH2 with hydrochloric acid. Extraction of the resulting aqueous fraction twice with ethyl acetate (250mL) and solvent removal gave 16g of product, which was used directly in the next reaction.

### Preparation of Formula I, where X is nitrogen, Y is carbon, R³ is chlorine, and R¹, R², R⁴, R⁵, R⁶ are hydrogen.

The material from the preceding reaction was mixed with 100mL of methanol. To this mixture was added 20mL of anhydrous hydrazine. The mixture was heated under reflux for 2 hours at which time an additional 100mL of methanol and 200mL of xylenes are added. The mixture was heated under reflux for an additional 17 hours. Additional xylenes (200mL) were added and 200mL of solvent was distilled. Isolation of the product was accomplished by cooling the reaction mixture and filtration. Washing of the resulting crystalline mass with toluene (200mL) and drying gave 14.2g of product.

### Preparation of Formula I, where X is nitrogen, Y is carbon, R¹ is 4-pyridylmethyl, R³ is chlorine, and R², R⁴, R⁵, R⁶ are hydrogen.

The material from the proceeding reaction (14.2g, 55mmoles) and sodium hydride (50% in oil, 2.7g, 55mmoles) were mixed in 150mL of l-methyl-2-pryrrolidone and the mixture was heated to 55°C. In a separatory funnel, 4-picolyl chloride hydrochloride [(4-chloromethyl)pyridine hydrochloride] (llg, 67mmoles), 50mL of saturated potassium carbonate and 150mL of toluene were mixed. After separation the toluene solution was added to the reaction mixture dropwise over the period of 30 minutes. The mixture was heated to 60°C for an additional 30 minutes, cooled to room temperature and poured into 300mL of water. The mixture was extracted twice with ethyl acetate (500mL). After drying the organic fraction with sodium sulfate (50g), filtering and solvent removal the crude product was crystallized from isopropyl acetate (300mL). The material was filtered and dried to yield 10.5 grams of product. An additional 4.6 grams of product was obtained as a second crop by concentration of the filtrates and filtering the resulting crystalline mass.

### Preparation of Formula I, where X is nitrogen, Y is carbon, R¹ is 4-pyridyl-N-oxide-methyl, R³ is chlorine, and R², R⁴, R⁵, R⁶ are hydrogen.

The product from the preceding reaction (6g, 17.2mmoles) was mixed with m-chloroperoxybenzoic acid (85%, 4.5g, 22.1mmoles) in methylene chloride (50mL). The mixture was stirred for 2 hours at room temperature. Saturated sodium carbonate (25mL) was added the resulting layers separated and the methylene chloride fraction dried with sodium sulfate (25g). After filtration, the solvent was removed and the resulting solid crystallized from methanol (20mL). Filtration and drying yielded 3.8g of product.

### EXAMPLE 42

### Preparation of 8-(3-chlorophenyl)-6-benzylpyrido-[2,3-d]pyridazin-5-thione.

**Conversion of a Pyrido[2,3-d]pyridazin-5-one of Formula I, where X is nitrogen, Y is carbon, Z is oxygen, R**^{**1**} **is benzyl, R**^{**3**} **is chloro, and R**^{**2**}**, R**^{**4**}**, R**^{**5**}**, R**^{**6**} **are hydrogen, into the corresponding Pyrido[2,3-d]-pyridazin-5-thione of Formula I, where X is nitrogen, Y is carbon and Z is sulfur.**

To a solution of 8-(3-chlorophenyl)-6-benzylpyrido[2,3-d]-pyridazin-5-one (0.96g, 2.76mmoles) in toluene (100mL) at room temperature under a nitrogen atmosphere was added solid 2,4-bis (4-methoxyphenyl)-1,3-dithia-2,4-diphosphetane-2,4-disulfide (Lawesson's Reagent) (0.67g, 1.66mmoles). The reaction mixture was heated under reflux for 18 hours, and then cooled and concentrated. Chromatography on silica gel using ethyl acetate/hexane (1:4) gave o.233g of product (23.2%), mp 132°C.

### EXAMPLE 43

### Preparation of 8-(3-chlorophenyl)-6-(6-methoxy-3-pyridyl-methyl)pyrido-[2,3-d] pyridazin-5-one.

### Formula I, where X is nitrogen, Y is carbon, Z is oxygen, R¹ is 6-methoxy-3-pyridylmethyl, R³ is chloro, and R², R⁴, R⁵, R⁶ are hydrogen.

To a suspension of 8-(3-chlorophenyl)pyrido[2,3-d]pyridazin-5-one (0.515g, 2.0 mmoles), 6-methoxy-3-hydroxymethyl pyridine (0.306g, 2.2 mmoles) and triphenylphosphine (0.792g, 3.0 mmoles) in THF (50mL) was added diisopropylazodicarboxylate (0.6mL, 3.0 mmoles). The reaction mixture was stirred for 18 hours at room temperature. The solvent was removed and the product was purified by chromatography (ethyl acetate/hexane 2:3) and crystallized from methanol to yield 0.588g of 8-(3-chlorophenyl)-6-(6-methoxy-3-pyridylmethyl)pyrido-[2,3-d]pyridazin-5-one, mp 145°C.

### EXAMPLE 44

### Preparation of 8-(3-chlorophenyl)-6-(3-pyridon-6-ylmethyl)pyrido[2,3-d]pyridazin-5-one.

### Formula I, where X is nitrogen, Y is carbon, Z is oxygen, R¹ is 3-pyridon-6-ylmethyl, R³ is chloro, and R², R⁴, R⁵, R⁶ are hydrogen.

To a solution of 8-(3-chlorophenyl)-6-(6-methoxy-3-pyridylmethyl)pyrido[2,3-d]pyridazin-5-one (0.566g, 1.5 mmoles) in acetonitrile was added solid sodium iodide (0.224g). The suspension was stirred for 30 min at room temperature, and then trimethylsilyl chloride (0.19mL, 1.5 mmoles) was added. The reaction mixture was heated under reflux for 18 hours, then allowed to cool. Water (50mL) was added. The solution was extracted with ethyl acetate, washed with 10% sodium thiosulfate and brine, dried over magnesium sulfate, filtered and concentrated. Chromatography with 10% methanol/ethyl acetate yielded 0.317g of 8-(3-chlorophenyl)-6-(3-pyridon-6-ylmethyl)pyrido-[2,3-d]-pyridazin-5-one, mp 233°C.

### EXAMPLE 45

### Preparation of 8-(3-chlorophenyl)-6-(1-methyl-3-pyridon-6-ylmethyl)-pyrido-[2,3-d]pyridazin-5-one.

### Formula I, where X is nitrogen, Y is carbon, Z is oxygen, R¹ is 1 methyl-3-pyridon-6-ylmethyl, R³ is chloro, and R², R⁴, R⁵, R⁶ are hydrogen.

To a suspension of 8-(3-chlorophenyl)-6-(3-pyridon-6-ylmethyl)pyrido-[2,3-d]pyridazin-5-one (0.35g, 0.96 mmoles) in DMF (15mL) was added potassium carbonate (0.16g, 1.15 mmoles). The suspension was stirred at room temperature for 1 hour under nitrogen, then methyl iodide (0.12mL) was slowly added via syringe. The reaction mixture was heated under reflux for 18 hours, then allowed to cool. 1N HCl (35mL) was added, the solid material collected, dried and chromatographed with ethyl acetate and then 5% methanol/ethyl acetate to give 0.055g of 8-(3-chlorophenyl)-6-(1-methyl-3-pyridon-6-ylmethyl)-pyrido[2,3-d]pyridazin-5-one, mp 171°C.

### EXAMPLE 46

### Determination of Potency and Selectivity of Inhibitors for PDE IV

### Preparation of Human Platelet Phosphodiesterase (PDE III)

Platelet high-affinity cAMP PDE (PDE III) was obtained from human blood in accordance with previously described procedures described in *Mol. Pharmacol.* **20**:302-309, Alvarez, R., Taylor, A., Fazarri, J.J., and Jacobs, J.R. (1981).

Blood was collected into evacuated tubes containing EDTA (7.7 mM, final concentration). PRP was obtained by centrifuging the blood in polycarbonate tubes at 200 x g for 15 min at 4°C. A platelet pellet was resuspended in a volume of buffer A (0.137 M NaCl, 12.3 mM Tris-HCl buffer, pH 7.7, containing 1 mM MgCl₂. The hypotonically-lysed platelet suspension was centrifuged at 48,000 x g for 15 min and the supernatant was saved. The pellets were frozen on dry ice and briefly thawed at 22°C. The supernatant was combined with the pellet fraction and the resulting suspension was centrifuged at 48,000 x g for 30 min. The supernatant fraction was stored in 0.5 ml aliquots at -20°C and used as the soluble PDE. Enzyme activity was adjusted to 10-20% hydrolysis after 10 minutes of incubation by dilution with 10mM cold Tris-HCl buffer, pH 7.7.

### Preparation of Human Lymphocyte Phosphodiesterase (PDE IV)

Human B cell line (43D) was cultured at 37°C in 7% CO₂ in RPMI 1640 with L-glutamine and 10% Nu-Serum. Prior to the assay ∼1.5x10⁸ cells were centrifuged at 1000 rpm for 10 minutes in a table top clinical centrifuge. The pellet was resuspended in 2-3 ml of 45 mM Tris-HCl buffer, pH 7.4. The suspension was homogenized and centrifuged at 12,000 x g 4°C for 10 minutes. The supernatant was diluted to 28 ml with Tris-HCl buffer and used directly in the assay or stored at -20°C. The final concentration of DMSO in the PDE incubation medium was 1%. Nitraquazone was included in each assay (10 and 100µM) as a reference standard.

### Human Platelet cAMP Phosphodiesterase Assay

The phosphodiesterase incubation medium contained 10 mM Tris-HCl buffer, pH 7.7, 10 mM MgSO₄, 0.1-1µM [³H]-AMP (0.2 µCi) in a total volume of 1.0 ml. Following addition of the enzyme, the contents were mixed and incubated for 10 min at 30°C. The reaction was terminated by immersing the tubes in a boiling-water bath for 90 sec. After the tubes were cooled in an ice-water bath, 0.1 ml (100µg) of 5'-nucleotidase from snake venom (Crotalus atrox, Sigma V-7000) was added to each tube. The contents were mixed and incubated for 30 min at 30°C. The nucleotidase reaction was terminated by immersing the tubes in a boiling water bath for 60 sec. Labeled adenosine was isolated from alumina columns according to the method described in *Anal. Biochem.,* **52**:505-516 (1973), Filburn, C.R., and Karn, J.. Assays were performed in triplicate. Hydrolysis of cAMP ranged from 10-20%. Test compounds were dissolved in DMSO. The final concentration of DMSO in the phosphodiesterase assay was 1% when tested with compounds up to 0.1 mM. When tested at 1 mM the DMSO concentration was 10% and this activity was compared to control PDE activity in the presence of 10% DMSO.

### Human Lymphocyte cAMP Phosphodiesterase Assay

The phosphodiesterase incubation medium contained 40 mM Tris-HCl buffer, pH 7.7, 0.1 mM MgSO4, 3.75 mM mercaptoethanol, and 0.1-1.0 µM [³H] CAMP (0.2 µCi) in a total volume of 1.0 ml. The reaction was performed and processed according to the procedure used (above) for human platelet PDE. The final concentration of DMSO was 1%.

The compounds of the present invention exhibit potency and selectivity as inhibitors of PDE IV when tested by this method.

| **Inhibition of Human Lymphocyte PDE IV** | | |
|---|---|---|
| Compound I | IC₅₀ | 0.00023 µM |
| Compound II | IC₅₀ | 0.001 µM |

**Compound I** is the compound of Formula I where X is nitrogen and Y is carbon, R¹ is 4-pyridylmethyl, R³ is nitro, R², R⁴, R⁵ and R⁶ are hydrogen, namely 6-(4-pyridylmethyl)-8-(3-nitrophenyl)pyrido[2,3-d]pyridazin-5-one.

**Compound II** is the compound of Formula I where X is nitrogen and Y is carbon, R¹ is 4-pyridylmethyl, R³ is chloro, R², R⁴, R⁵ and R⁶ are hydrogen, namely 6-(4-pyridylmethyl)-8-(3-chlorophenyl)pyrido[2,3-d]pyridazin-5-one.

### EXAMPLE 47

### Determination of Immunosuppressive Activity Utilizing The Hemolytic Plaque Forming Cell (PFC) Assay

This procedure is a modification of "The agar plaque technique for recognizing antibody producing cells," a procedure initially described by Jerne, et al. [*Cell-bound Antibodies,* Amos and Kaprowski editors (Wistar Institute Press, Philadelphia, 1963) p. 109].

Groups of 5-6 adult C3H female mice were sensitized with 1.25 x 10⁸ sheep red blood cells (SRBC) and simultaneously treated with an oral dosage form of the test material in an aqueous vehicle. Animals in a control group receive the same volume of vehicle. Four days after SRBC inoculation, spleens are dispersed in glass homogenizers. The number of nucleated cells (WBC) is determined and the spleen cell suspension is mixed with SRBC, guinea pig complement and agar solution at 0.5% concentration. Aliquots of the above mixture (0.1 ml) are dropped on four separate quadrants of a Petri dish and are covered with cover slips. After two hours incubation at 37°C, areas of hemolysis around plaque-forming cells (PFC) are counted with a dissecting microscope. Total WBC/spleen, PFC/spleen and PFC/10⁶ WBC (PPM) are calculated for each mouse spleen. Geometric means of each treatment group are then compared with the vehicle-treated control group.

The compounds of the present invention show immunosuppressive activity when tested by this method.

| **Immunosuppressive Activity of Compounds I and II** | | |
|---|---|---|
| | Immune PFC | |
| Compound I | ED₅₀ | 1 mg/kg |
| Compound II | ED₅₀ | 3 mg/kg |

### EXAMPLE 48

### Determination of Immunosuppressive Activity Utilizing Responses of Human Peripheral Blood Lymphocytes to Mitogen

This procedure is a modification of a procedure initially described by Greaves, et al. ["Activation of human T and B lymphocytes by polyclonal mitogens," *Nature,* 248, 698-701 (1974)].

Human mononuclear cells (PBL) are separated from heparinized whole blood by density gradient centrifugation in Ficoll-Paque (Pharmacia). After washing, 5 x 10⁴ cells/well are cultured in microtiter plates with minimal essential media supplemented with 1% human serum, gentamicin, sodium bicarbonate, 2-mercaptoethanol, glutamine, non-essential amino acids, and sodium pyruvate. The mitogen concanavalin A (Sigma) is used at a concentration of 2 µg/ml. Test materials are tested at concentrations between 10⁻⁴ and 10⁻¹⁰ M, by addition to the culture at time 0. Cultures are set up in quadruplicate and incubated at 37°C in a humidified atmosphere with 5% CO₂ for 48 hours. A pulse of 1.0 µCi/well of ³H-thymidine is added for the last 4 hours. Cells are collected on glass fiber filters with an automatic harvester and radioactivity is measured by standard scintillation procedures. The 50% inhibitory concentration ("IC₅₀") for mitogenic stimulation is determined graphically. The compounds of the present invention show immunosuppressive activity when tested by this method.

| **Effect of Compounds on Mitogenic Response of Human PBL to Concanavalin in a Stimulation** | | |
|---|---|---|
| **Concentration** | **% Suppression** | |
| | Compound I | Compound II |
| 100 µM | 100 | 100 |
| 10 µM | 85 | 87 |
| 1 µM | 80 | 83 |
| 0.1 µM | 83 | 74 |

The % suppression represents test well counts minus background (wells with no concanavalin A) divided by control well counts (wells with no compound) minus background times 100.

### EXAMPLE 49

### Determination of Anti-Inflammatory Activity Utilizing Arachidonic Acid-Induced Ear Edema (AAEE) in the Mouse

This procedure is a modification of a procedure described by Young et al., *J. Invest. Derm.,* 82:367-371 (1984).

Female Charles River ICR mice 23-27 grams are administered 0.2 ml of test material. The mice are later challenged with 20 µl of arachidonic acid applied topically to the ear. One hour after challenge, the weight of an 8 mm disc is determined. The mean increase in ear plug weight is calculated. Materials with anti-inflammatory activity inhibit the increase in ear plug weight.

The compounds of the present invention exhibit anti-inflammatory activity when tested by this method.

| **Antiinflammatory Activity of Compounds I and II** | | |
|---|---|---|
| | AAEE | |
| Compound I | ED₅₀ | 0.003mg/kg |
| Compound II | ED₅₀ | 0.009mg/kg |

### EXAMPLE 50

### Capsule Formulation

This example illustrates the preparation of a representative pharmaceutical formulation for oral administration containing an active compound of Formula I.

| **Ingredients** | **Quantity (mg/capsule)** |
|---|---|
| Active compound | 200 mg |
| lactose, spray-dried | 148 mg |
| magnesium stearate | 2 mg |

The above ingredients are mixed and introduced into a hard-shell gelatin capsule.

### EXAMPLE 51

### Oral Formulation

This example illustrates the preparation of a representative pharmaceutical formulation containing an active compound of Formula I.

An suspension for oral administration is prepared having the following composition:

| **Ingredients** | **Quantity** |
|---|---|
| Active compound | 1.0 g |
| fumaric acid | 0.5 g |
| sodium chloride | 2.0 g |
| methyl paraben | 0.1 g |
| granulated sugar | 25.5 g |
| sorbitol (70% solution) | 12.85 g |
| Veegum K (Vanderbilt Co.) | 1.0 g |
| flavoring | 0.035 mL |
| colorings | 0.5 mg |
| distilled water | q.s. to 100 mL |

Other compounds of Formula I, such as those prepared in accordance with Examples 1-45 can be used as the active compound in the preparation of the orally administrable formulations of this example.

### EXAMPLE 52

### Tablet Formulation

This example illustrates the preparation of a representative pharmaceutical formulation containing an active compound of Formula I.

A tablet for oral administration is prepared having the following composition:

| **Ingredients** | **Quantity (mg/tablet)** |
|---|---|
| Active compound | 400 mg |
| corn starch | 50 mg |
| lactose | 145 mg |
| magnesium stearate | 5 mg |

The above ingredients are mixed intimately and pressed into single scored tablets.

Other compounds of Formula I, such as those prepared in accordance with Examples 1-45 can be used as the active compound in the preparation of the tablet formulations of this example.

### EXAMPLE 53

### Injectable Formulation

This example illustrates the preparation of a representative pharmaceutical formulation containing an active compound of Formula I.

An injectable preparation is prepared having the following composition:

| **Ingredients** | **Quantity** |
|---|---|
| Active compound | 200 mg |
| water (distilled, sterile) | q.s. to 20.0 mL |

Other compounds of Formula I, such as those prepared in accordance with Examples 1-45 can be used as the active compound in the preparation of the injection administrable formulations of this example.

### EXAMPLE 54

### Suppository Formulation

This example illustrates the preparation of a representative pharmaceutical formulation containing an active compound of Formula I.

A suppository totalling 2.5 grams is prepared having the following composition:

| **Ingredients** | **Quantity** |
|---|---|
| Active compound | 0.5 g |
| Witepsol H-15* | q.s. to 2.5 g |

| | |
|---|---|
| (*triglycerides of saturated vegetable fatty acid; a product of Riches-Nelson, Inc., New York, N.Y.). | |

Other compounds of Formula I, such as those prepared in accordance with Examples 1-45 can be used as the active compound in the preparation of the suppository formulations of this example.

Other compounds of Formula I, such as those prepared in accordance with Examples 1-45 can be used as the active compound in the preparation of the injection administrable formulations of this example.

| **TOXICOLOGY** | |
|---|---|
| SPECIES: | mice |
| COMPOUND: | compounds I, II |
| DOSAGE; | 3 to 25 mg/kg/day |
| DURATION OF DOSING: | 2 weeks |
| CONCLUSION: | no mortality occurred at any of the dose levels tested. |

**The Ames test** (in vitro Salmonella mutagenicity assay) **was negative.**

## Claims

1. A compound of the formula: 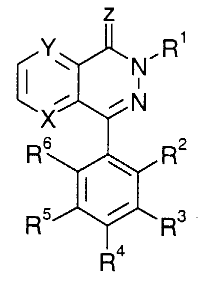 wherein:
X and Y are carbon or nitrogen, provided both are not the same;
Z is oxygen or sulfur;
R¹ is hydrogen; phenyl or naphthyl, optionally mono or disubstituted with hydroxy, C₁₋₉ alkyl, C₁₋₉ alkoxy, trifluoromethyl, nitro or cyano; 5-or 6-membered heteroaryl containing 1 to 3 heteroatoms; 4-pyridylmethyl, 3-pyridylethyl, 2-pyridylmethyl, 2-pyridylpropyl, 3-thienylmethyl or 2-thienylethyl.
R², R³, R⁴, R⁵ and R⁶ are independently selected from hydrogen, C₁₋₉ alkyl, halo, carboxy, C₁₋₉ alkoxycarbonyl, methylthio, trifluoromethyl, cyano, nitro and carbamoyl of the formula -C(O)NR'R" where R' and R" are independently hydrogen or C₁₋₉ alkyl;
or a pharmaceutically acceptable ester or salt thereof.

2. The compound of Claim 1 wherein X is nitrogen, Y is carbon and Z is oxygen.

3. The compound of Claim 2 wherein R³ is hydrogen, halo or nitro and R², R⁴, R⁵ and R⁶ are hydrogen.

4. The compound of Claim 3 wherein R¹ is hydrogen; phenyl, 5- or 6-membered heteroaryl containing 1 to 3 heteroatoms; 4-pyridylmethyl, 3-pyridylethyl, 2-pyridylmethyl, 2-pyridylpropyl, 3-thienylmethyl or 2-thienylethyl.

5. The compound of Claim 4 wherein R¹ is 4-pyridylmethyl, 3-pyridylmethyl or 3-thienylmethyl.

6. The compound of Claim 1 wherein X is carbon, Y is nitrogen and Z is oxygen.

7. The compound of Claim 6 wherein R¹ is hydrogen, phenyl 5- or 6-membered heteroaryl containing 1 to 3 heteroatoms; or 4-pyridylmethyl, 3-pyridylethyl, 2-pyridylmethyl, 2-pyridylpropyl, 3-thienylmethyl or thienylethyl, and R³ is hydrogen, halo or nitro and R², R⁴, R⁵ and R⁶ are hydrogen.

8. The compound of Claim 1 wherein X is nitrogen, Y is carbon and Z is sulfur.

9. The compound of Claim 8 wherein R³ is hydrogen, halo or nitro and R², R⁴, R⁵ and R⁶ are hydrogen.

10. A compound of the formula: 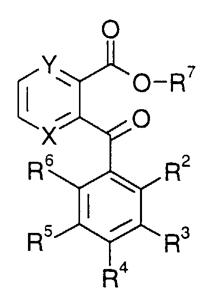 wherein:
X, Y, R², R³, R⁵, R⁶ are as defined in claim 1,
R⁴ is hydrogen, C₁₋₉ alkyl, iodo, bromo, chloro, carboxy, C₁₋₉ alkyl carbonyl, nitro or carbamoyl of the formula -C(O)NR'R" where R' and R" are independently hydrogen of C₁₋₉ alkyl; and
R⁷ is C₁₋₉ alkyl; provided that when X is carbon, Y is nitrogen and R⁷ is methyl, R², R³, R⁴, R⁵ and R⁶ are not all hydrogen;
or a pharmaceutically acceptable ester or salt thereof.

11. The compound of Claim 10 wherein R³ is hydrogen, halo or nitro.

12. A pharmaceutical composition comprising a pharmaceutically acceptable excipient and a therapeutically effective amount of a compound of the formula 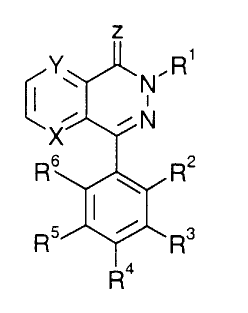 wherein: X, Y, Z, R¹, R², R³, R⁴, R⁵ and R⁶ are as defined in claim 1
or a pharmaceutically acceptable ester or salt thereof.

13. Use of a compound represented by the formula 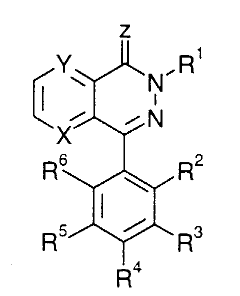 wherein: X, Y, Z, R¹, R², R³, R⁴, R⁵ and R⁶ are as defined in claim 1
or a pharmaceutically acceptable ester or salt thereof, in the manufacture of a medicament for treating asthma, pain, inflammatory disease, allograft rejection, graft-vs-host-rejection, and autoimmune disease in mammals.

14. A process for making a compound of the formula (I) 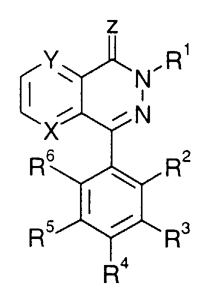 wherein: X, Y, Z, R¹, R², R³, R⁴, R⁵ and R⁶ are as defined in claim 1 or a pharmaceutically acceptable ester or salt thereof, which comprises
(a) reacting a compound of the formula (II) 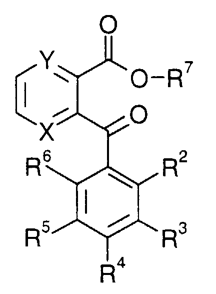 wherein:
X and Y are carbon or nitrogen, provided both are not the same;
R², R³, R⁵ and R⁶ are independently selected from hydrogen, C₁₋₉ alkyl, halo, carboxy, C₁₋₉ alkoxycarbonyl, methylthio, trifluoromethyl, cyano, nitro and carbamoyl of the formula -C(O)NR'R" where R' and R" are independently hydrogen or C₁₋₉ alkyl;
R⁴ is hydrogen, C₁₋₉ alkyl, iodo, bromo, chloro, carboxy, C₁₋₉ alkyl carbonyl, nitro or carbamoyl of the formula -C(O)NR'R" where R' and R" are independently hydrogen or C₁₋₉ alkyl; and
R⁷ is C₁₋₉ alkyl,
with a compound of the formula 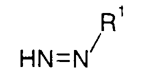 wherein:
R¹ is hydrogen; phenyl, naphthyl, optionally mono or disubstituted with hydroxy, C₁₋₉ alkyl, C₁₋₉ alkoxy, halo, trifluoromethyl, nitro or cyano; 5- or 6-membered heteroaryl containing containing 1 to 3 heteroatoms; or 4-pyridylmethyl, 3-pyridylethyl, 2-pyridylmethyl, 2-pyridylpropyl, 3-thienylmethyl or 2-thienylethyl;
(b) converting a compound of the formula (I) to a pharmaceutically acceptable salt of the formula (I); or
(c) converting a salt of a compound of the formula (I) to the corresponding free base or to another pharmaceutically acceptable salt of the formula (I).

15. The process of claim 14 further comprising the step of treating a compound of formula I wherein Z is oxygen with a thiation reagent to produce a compound of formula I wherein Z is sulfur.

## Patentansprüche

1. Verbindung der Formel: 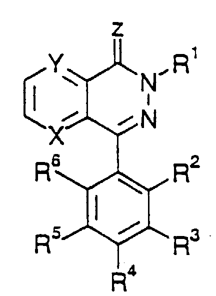 worin:
X und Y Kohlenstoff oder Stickstoff sind, mit der Maßgabe, daß beide nicht gleich sind;
Z Sauerstoff oder Schwefel ist;
R¹ Wasserstoff; Phenyl oder Naphthyl, gegebenenfalls mono- oder disubstituiert mit Hydroxy, C₁₋₉-Alkyl, C₁₋₉-Alkoxy, Trifluormethyl, Nitro oder Cyano; 5- oder 6-gliedriges Heteroaryl mit 1 bis 3 Heteroatomen; 4-Pyridylmethyl, 3-Pyridylethyl, 2-Pyridylmethyl, 2-Pyridylpropyl, 3-Thienylmethyl oder 2-Thienylethyl ist;
R², R³, R⁴, R⁵ und R⁶ unabhängig ausgewählt sind aus Wasserstoff, C₁₋₉-Alkyl, Halogen, Carboxy, C₁₋₉-Alkoxycarbonyl, Methylthio, Trifluormethyl, Cyano, Nitro und Carbamoyl der Formel -C(O)NR'R", wobei R' und R" unabhängig Wasserstoff oder C₁₋₉-Alkyl sind;
oder ein pharmazeutisch annehmbarer Ester oder ein pharmazeutisch annehmbares Salz davon.

2. Verbindung nach Anspruch 1, worin X Stickstoff, Y Kohlenstoff und Z Sauerstoff ist.

3. Verbindung nach Anspruch 2, worin R³ Wasserstoff, Halogen oder Nitro ist und R², R⁴, R⁵ und R⁶ Wasserstoff sind.

4. Verbindung nach Anspruch 3, worin R¹ Wasserstoff, Phenyl, 5- oder 6-gliedriges Heteroaryl mit 1 bis 3 Heteroatomen, 4-Pyridylmethyl, 3-Pyridylethyl, 2-Pyridylmethyl, 2-Pyridylpropyl, 3-Thienylmethyl oder 2-Thienylethyl ist.

5. Verbindung nach Anspruch 4, worin R¹ 4-Pyridylmethyl, 3-Pyridylmethyl oder 3-Thienylmethyl ist.

6. Verbindung nach Anspruch 1, worin X Kohlenstoff, Y Stickstoff und Z Sauerstoff ist.

7. Verbindung nach Anspruch 6, worin R¹ Wasserstoff, Phenyl, 5- oder 6-gliedriges Heteroaryl mit 1 bis 3 Heteroatomen, oder 4-Pyridylmethyl, 3-Pyridylethyl, 2-Pyridylmethyl, 2-Pyridylpropyl, 3-Thienylmethyl oder Thienylethyl ist, und R³ Wasserstoff, Halogen oder Nitro ist und R², R⁴, R⁵ und R⁶ Wasserstoff sind.

8. Verbindung nach Anspruch 1, worin X Stickstoff, Y Kohlenstoff und Z Schwefel ist.

9. Verbindung nach Anspruch 8, worin R³ Wasserstoff, Halogen oder Nitro ist und R², R⁴, R⁵ und R⁶ Wasserstoff sind.

10. Verbindung der Formel: 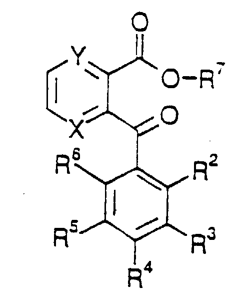 worin:
X, Y, R², R³, R⁵, R⁶ wie in Anspruch 1 definiert sind,
R⁴ Wasserstoff, C₁₋₉-Alkyl, lod, Brom, Chlor, Carboxy, C₁₋₉-Alkylcarbonyl, Nitro oder Carbamoyl der Formel -C(O)NR'R" ist, wobei R' und R" unabhängig Wasserstoff oder C₁₋₉-Alkyl sind; und
R⁷ C₁₋₉-Alkyl ist; mit der Maßgabe, daß wenn X Kohlenstoff, Y Stickstoff und R⁷ Methyl ist, R², R³, R⁴, R⁵ und R⁶ nicht alle Wasserstoff sind;
oder ein pharmazeutisch annehmbarer Ester oder ein pharmazeutisch annehmbares Salz davon.

11. Verbindung nach Anspruch 10, worin R³ Wasserstoff, Halogen oder Nitro ist.

12. Pharmazeutische Zusammensetzung, umfassend einen pharmazeutisch annehmbaren Exzipienten und eine therapeutisch wirksame Menge einer Verbindung der Formel 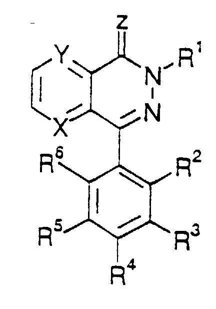 worin X, Y, Z, R¹, R², R³, R⁴, R⁵ und R⁶ wie in Anspruch 1 definiert sind, oder eines pharmazeutisch annehmbaren Esters oder Salzes davon.

13. Verwendung einer Verbindung, welche durch die Formel 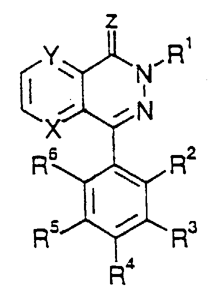 dargestellt wird, worin X, Y, Z, R¹, R², R³, R⁴, R⁵ und R⁶ wie in Anspruch 1 definiert sind,
oder eines pharmazeutisch annehmbaren Esters oder Salzes davon zur Herstellung eines Medikaments zur Behandlung von Asthma, Schmerzen, Entzündungserkrankung, Allograft-Abstoßung, Transplantat-Wrt-Abstoßung und Autoimmunerkrankung bei Säugern.

14. Verfahren zur Herstellung einer Verbindung der Formel (I) 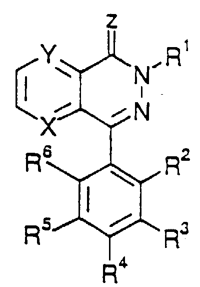 worin X, Y, Z, R¹, R², R³, R⁴, R⁵ und R⁶ wie in Anspruch 1 definiert sind,
oder eines pharmazeutisch annehmbaren Esters oder Salzes davon, welches umfaßt
(a) Umsetzung einer Verbindung der Formel (II) 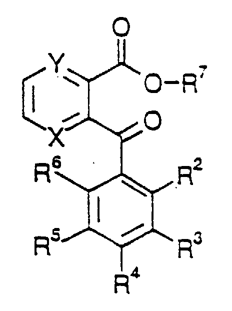 worin:
X und Y Kohlenstoff oder Stickstoff sind, mit der Maßgabe, daß beide nicht gleich sind;
R², R³, R⁵ und R⁶ unabhängig ausgewählt sind aus Wasserstoff, C₁₋₉-Alkyl, Halogen, Carboxy, C₁₋₉-Alkoxycarbonyl, Methylthio, Trifluormethyl, Cyano, Nitro und Carbamoyl der Formel -C(O)NR'R", wobei R' und R" unabhängig Wasserstoff oder C₁₋₉-Alkyl sind;
R⁴ Wasserstoff, C₁₋₉-Alkyl, Iod, Brom, Chlor, Carboxy, C₁₋₉-Alkylcarbonyl, Nitro oder Carbamoyl der Formel -C(O)NR'R" ist, wobei R' und R" unabhängig Wasserstoff oder C₁₋₉-Alkyl sind; und
R⁷ C₁₋₉-Alkyl ist,
mit einer Verbindung der Formel 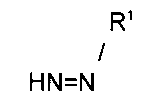 worin:
R¹ Wasserstoff; Phenyl, Naphthyl, gegebenenfalls mono- oder disubstituiert mit Hydroxy, C₁₋₉-Alkyl, C₁₋₉-Alkoxy, Halogen, Trifluormethyl, Nitro oder Cyano; 5- oder 6-gliedriges Heteroaryl mit 1 bis 3 Heteroatomen; oder 4-Pyridylmethyl, 3-Pyridylethyl, 2-Pyridylmethyl, 2-Pyridylpropyl, 3-Thienylmethyl oder 2-Thienylethyl ist;
(b) Überführung einer Verbindung der Formel (I) in ein pharmazeutisch annehmbares Salz der Formel (I); oder
(c) Überführung eines Salzes einer Verbindung der Formel (I) in die entsprechende freie Base oder in ein anderes pharmazeutisch annehmbares Salz der Formel (I).

15. Verfahren nach Anspruch 14, umfassend femer den Schritt der Behandlung einer Verbindung der Formel (I), worin Z Sauerstoff ist, mit einem Thiations-Reagenz, um eine Verbindung der Formel (I) zu erzeugen, worin Z Schwefel ist.

## Revendications

1. Composé de formule : 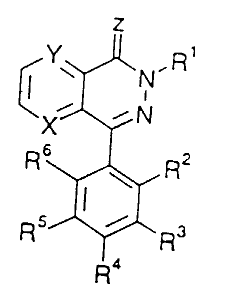 dans laquelle :
X et Y représentent le carbone ou l'azote, sous réserve que X et Y ne soient pas identiques ;
Z représente l'oxygène ou le soufre ;
R¹ représente l'hydrogène ; un groupe phényle ou naphtyle, facultativement mono- ou disubstitué avec des substituants hydroxy, alkyle en C₁ à C₉, alkoxy en C₁ à C₉, trifluorométhyle, nitro ou cyano ; un groupe hétéroaryle penta- ou hexagonal contenant 1 à 3 hétéroatomes ; un groupe 4-pyridylméthyle, 3-pyridyléthyle, 2-pyridylméthyle, 2-pyridylpropyle, 3-thiénylméthyle ou 2-thiényléthyle ;
R², R³, R⁴, R⁵ et R⁶ sont choisis, indépendamment, entre l'hydrogène, des groupes alkyle en C₁ à C₉, halogéno, carboxy, (alkoxy en C₁ à C₉)carbonyle, méthylthio, trifluorométhyle, cyano, nitro et carbamoyle de formule -C(O)NR'R" dans laquelle R' et R" représentent, indépendamment, l'hydrogène ou un groupe alkyle en C₁ à C₉ ;
ou un de ses esters ou sels pharmaceutiquement acceptables.

2. Composé suivant la revendication 1, dans lequel X représente l'azote, Y représente le carbone et Z représente l'oxygène.

3. Composé suivant la revendication 2, dans lequel R³ représente l'hydrogène, un groupe halogéno ou nitro et R², R⁴, R⁵ et R⁶ représentent l'hydrogène.

4. Composé suivant la revendication 3, dans lequel R¹ représente l'hydrogène ; un groupe phényle, hétéroaryle penta- ou hexagonal contenant 1 à 3 hétéroatomes ; 4-pyridylméthyle, 3-pyridyléthyle, 2-pyridylméthyle, 2-pyridylpropyle, 3-thiénylméthyle ou 2-thiényléthyle.

5. Composé suivant la revendication 4, dans lequel R¹ représente un groupe 4-pyridylméthyle, 3-pyridylméthyle ou 3-thiénylméthyle.

6. Composé suivant la revendication 1, dans lequel X représente le carbone, Y représente l'azote et Z représente l'oxygène.

7. Composé suivant la revendication 6, dans lequel R¹ représente l'hydrogène, un groupe phényle, hétéroaryle penta- ou hexagonal contenant 1 à 3 hétéroatomes ; ou 4-pyridylméthyle, 3-pyridyléthyle, 2-pyridylméthyle, 2-pyridylpropyle, 3-thiénylméthyle ou thiényléthyle, et R³ représente l'hydrogène, un groupe halogéno ou nitro, et R², R⁴, R⁵ et R⁶ représentent l'hydrogène.

8. Composé suivant la revendication 1, dans lequel X représente l'azote, Y représente le carbone et Z représente le soufre.

9. Composé suivant la revendication 8, dans lequel R³ représente l'hydrogène, un groupe halogéno ou nitro et R², R⁴, R⁵ et R⁶ représentent l'hydrogène.

10. Composé de formule : 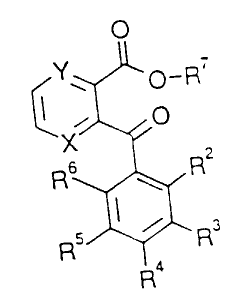 dans laquelle :
X, Y, R², R³, R⁵ et R⁶ répondent aux définitions suivant la revendication 1,
R⁴ représente l'hydrogène, un groupe alkyle en C₁ à C₉, iodo, bromo, chloro, carboxy, (alkyle en C₁ à C₉)-carbonyle, nitro ou carbamoyle de formule -C(O)NR'R" dans laquelle R' et R" représentent, indépendamment, l'hydrogène ou un groupe alkyle en C₁ à C₉ ; et
R⁷ représente un groupe alkyle en C₁ à C₉ ; sous réserve que, lorsque X représente le carbone, Y représente l'azote et R⁷ représente un groupe méthyle, R², R³, R⁴, R⁵ et R⁶ ne représentent pas tous l'hydrogène ;
ou un de ses esters ou sels pharmaceutiquement acceptables.

11. Composé suivant la revendication 10, dans lequel R³ représente l'hydrogène, un groupe halogéno ou nitro.

12. Composition pharmaceutique comprenant un excipient pharmaceutiquement acceptable et une quantité thérapeutiquement efficace d'un composé de formule 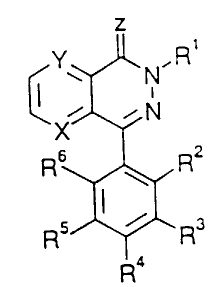 dans laquelle :
X, Y, Z, R¹, R², R³, R⁴, R⁵ et R⁶ répondent aux définitions suivant la revendication 1,
ou d'un de ses esters ou sels pharmaceutiquement acceptables.

13. Utilisation d'un composé représenté par la formule 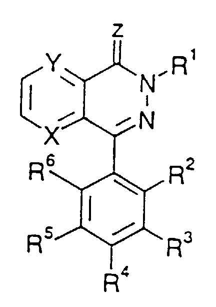 dans laquelle :
X, Y, Z, R¹, R², R³, R⁴, R⁵ et R⁶ répondent aux définitions suivant la revendication 1,
ou d'un de ses esters ou sels pharmaceutiquement acceptables, dans la production d'un médicament destiné au traitement de l'asthme, de la douleur, d'une maladie inflammatoire, d'un rejet d'allogreffe, d'une réaction du greffon contre l'hôte et d'une maladie auto-immune chez des mammifères.

14. Procédé pour la préparation d'un composé de formule (I) 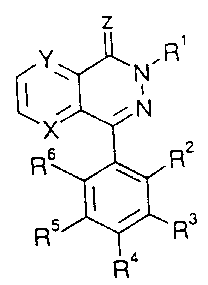 dans laquelle :
X, Y, Z, R¹, R², R³, R⁴, R⁵ et R⁶ répondent aux définitions suivant la revendication 1,
ou d'un de ses esters ou sels pharmaceutiquement acceptables, qui comprend
(a) la réaction d'un composé de formule (II) 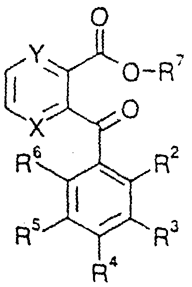 dans laquelle :
X et Y représentent le carbone ou l'azote, sous réserve que X et Y ne soient pas identiques ;
R², R³, R⁵ et R⁶ sont choisis, indépendamment, entre l'hydrogène, des groupes alkyle en C₁ à C₉, halogéno, carboxy, (alkoxy en C₁ à C₉) carbonyle, méthylthio, trifluorométhyle, cyano, nitro et carbamoyle de formule -C(O)NR'R" dans laquelle R' et R" représentent, indépendamment, l'hydrogène ou un groupe alkyle en C₁ à C₉ ;
R⁴ représente l'hydrogène, un groupe alkyle en C₁ à C₉, iodo, bromo, chloro, carboxy, (alkyle en C₁ à C₉)-carbonyle, nitro ou carbamoyle de formule -C(O)NR'R" dans laquelle R' et R" représentent, indépendamment, l'hydrogène ou un groupe alkyle en C₁ à C₉ ; et
R⁷ représente un groupe alkyle en C₁ à C₉,
avec un composé de formule 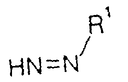 dans laquelle :
R¹ représente l'hydrogène ; un groupe phényle, naphtyle, facultativement mono- ou disubstitué avec des substituants hydroxy, alkyle en C₁ à C₉, alkoxy en C₁ à C₉, halogéno, trifluorométhyle, nitro ou cyano ; un groupe hétéroaryle penta- ou hexagonal contenant 1 à 3 hétéroatomes ; ou un groupe 4-pyridylméthyle, 3-pyridyléthyle, 2-pyridylméthyle, 2-pyridylpropyle, 3-thiénylméthyle ou 2-thiényléthyle ;
(b) la transformation d'un composé de formule (I) en un sel pharmaceutiquement acceptable de ce composé de formule (I) ; ou
(c) la transformation d'un composé de formule (I) en la base libre correspondante ou en un autre sel pharmaceutiquement acceptable de ce composé de formule (I).

15. Procédé suivant la revendication 14, comprenant en outre l'étape consistant à traiter un composé de formule I dans laquelle Z représente l'oxygène avec un réactif de sulfuration pour produire un composé de formule I dans laquelle Z représente le soufre.
